# EUROPEAN PATENT APPLICATION

(11) **EP 4 450 515 A1**
(43) Date of publication of application: **23.10.2024**
(21) Application number: 22906707.9
(22) Date of filing: 16.12.2022
(51) Int. Cl.: C07K 14/31, C07K 16/00, C07K 1/22, C12N 15/31, C12N 15/62, B01D 15/38

(54) **DOMAIN C OF MUTATED PROTEIN A, AND APPLICATION THEREOF**

(30) Priority: 17.12.2021 CN 202111555288
(71) Applicant: Bestchrom (Shanghai) Biosciences Ltd., Shanghai 201203 (CN); Bestchrom (Zhejiang) Biosciences Ltd., Zhejiang 314299 (CN)
(72) Inventor: ZHANG, Hong, Zhejiang 314299 (CN); WU, Xiaojun, Zhejiang 314299 (CN); HE, Wenxia, Zhejiang 314299 (CN); MA, Zhenzhen, Zhejiang 314299 (CN)
(74) Representative: V.O.
(86) International application number: PCT/CN2022/139746
(87) International publication number: WO 2023/109963

(57) **Abstract**

The present invention relates to domain C of mutated protein A, and an application thereof. The present invention specifically provides a separated polypeptide. The polypeptide has a substitution mutation as described herein at one or more positions selected from positions 3, 6, 9 and 15 compared with the natural C-domain of the protein A shown in SEQ ID NO: 1. The present invention also provides a fusion protein comprising the polypeptide, a recombinant protein A, a corresponding coding sequence, a nucleic acid construct, an expression system, a separation matrix, a method, and use. Compared with the natural C-domain, the separated polypeptide in the present invention has an obviously improved alkaline stability in an alkali cleaning process.

## Description

### Technical Field

The present description relates to a mutated domain C of protein A, and an application thereof.

### Technical Background

In recent years, antibody drugs are gaining increasing popularity in the global pharmaceutical market due to their high specificity. Monoclonal antibody is one of the fastest growing fields in the biopharmaceutical industry. Monoclonal antibodies possess three unique mechanisms of action, specifically, targeting effect, blocking effect and signal transduction effect. Monoclonal antibody drugs are mainly used in the treatment of tumors, autoimmune diseases and infectious diseases, with an exceptional efficacy in cancer treatment.

At present, the purification of antibody drugs mainly relies on the capture of antibodies by the special adsorption capacity of multiple regions of the affinity resin ligand protein A to the unique segment of antibody Fc. This method is the most widely used affinity chromatography methods based on mature technology.

However, the protein A ligand may shed from the protein A affinity chromatography resin during the antibody purification process. The shed ligand needs to be removed from the process to avoid drug immune reaction. The shedding rate of the ligand largely depends on the amino acid distribution, stability and coupling pattern of the ligand. Meanwhile, for cost control reason, the chromatographic medium is often used repeatedly, which means a cleaning process is required to restore the resin to pre-use state. Usually, it can be cleaned with 0.1-0.5M standard NaOH solution (CIP) to efficiently remove protein precipitates, hydrophobic proteins, nucleic acids, endotoxins, viruses, etc. from the chromatographic medium. However, when being cleaned by alkaline solution, the carrier will be exposed to alkaline conditions. For many chromatography media containing protein affinity ligands, such environment is very undesirable for the possible damage to the amino acids of ligands, and therefore adversely affect the adsorption capacity and life cycle of the medium.

Therefore, overcoming the challenge of unstable protein A affinity chromatography medium in alkaline environment and enabling its wide use in the field of biological affinity chromatography has become a key target.

### Summary of the Description

The present description provides a mutant polypeptide of the C domain of the native protein A, having improved alkaline stability compared with the C domain of the native protein A.

Specifically, the present description provides a polypeptide, compared with the native C domain of the protein A shown in SEQ ID NO: 1, the polypeptide has substitution mutations at one or more positions selected from the group consisting of positions 3, 6, 9 and 15.

In one or more embodiments, compared with the native C domain of the protein A shown in SEQ ID NO: 1, the polypeptide has substitution mutations at the positions 3, 6, 9 or 15; In some embodiments, compared with the native C domain of the protein A shown in SEQ ID NO: 1, the polypeptide has substitution mutations at the positions 3 and 6, or at the positions 3 and 9, or at the positions 3 and 15, or at the positions 6 and 9, or at the positions 6 and 15, or at the positions 9 and 15; In some embodiments, compared with the native C domain of the protein A shown in SEQ ID NO: 1, the polypeptide has substitution mutations at the positions 3, 6 and 9, or at the positions 3, 6 and the 15, or at the positions 6, 9 and 15; In some embodiments, compared with the native C domain of the protein A shown in SEQ ID NO: 1, the polypeptide has substitution mutations at the positions 3, 6, 9 and 15.

In one or more embodiments, compared with the native C domain of the protein A shown in SEQ ID NO: 1, the polypeptide has a substitution mutation at the position 15, and optionally has substitution mutations at one or more positions selected from the positions 3, 6 and 9.

In one or more embodiments, compared with the native C domain of the protein A shown in SEQ ID NO: 1, the polypeptide has substitution mutations at the position 9 and 15, and optionally has substitution mutations at one or all two positions selected from the positions 3 and 6.

In one or more embodiments, compared with the native C domain of the protein A shown in SEQ ID NO: 1, the polypeptide has substitution mutations at the positions 3, 9 and 15, and optionally has a substitution mutation at the position 6.

In one or more embodiments, the substitution mutation at the position 3 is N3L, N3V or N3Y.

In one or more embodiments, the substitution mutation at the position 6 is N6S, N6L or N6E.

In one or more embodiments, the substitution mutation at the position 9 is Q9I, Q9F or Q9M.

In one or more embodiments, the substitution mutation at the position 15 is E15T, E15W, E15L, E15V, E151, E15F, E15S, E15Y or E15T.

In one or more embodiments, the amino acid sequence of the polypeptide is as shown in any one of SEQ ID NO: 2-73.

In one or more embodiments, compared with the native C domain of the protein A shown in SEQ ID NO: 1, the amino acid residue at the position 29 of the polypeptide is A.

The second aspect of the present description provides a polypeptide, which consists of the following (i), (ii) and (iii): (i) the polypeptide described in any embodiment of the first aspect of the present description, (ii) one or more coupling elements at the C-terminus or N-terminus of the amino acid sequence of the polypeptide (i), and optionally (iii) residues from the excised signal transduction sequence.

In one or more embodiments, the coupling element is selected from the group consisting of: cysteine residues, multiple lysine residues and multiple histidine residues.

The third aspect of the present description provides a fusion protein, which is fused by 2-8 polypeptides, wherein, compared with the native C domain of the protein A shown in SEQ ID NO: 1, the amino acid sequence of the polypeptide has substitution mutations at one or more positions selected from the group consisting of positions 3, 6, 9 and 15.

In one or more embodiments, the polypeptide is a polypeptide described in any embodiment of the first aspect of the present description.

In one or more embodiments, in the fusion protein, the 2-8 polypeptides are different, partially or completely identical to each other.

In one or more embodiments, each polypeptide contained in the fusion protein contains at least E15T, E15W, E15L, E15V, E15I, E15F, E15S, E15Y or E15T mutations, and preferably also containing one or more substitution mutations at the positions 3, 6, 9 as described in the present description, preferably the substitution mutation at the position 3 is N3L or N3V, preferably the substitution mutation at the position 6 is N6S, N6L or N6E, preferably the substitution mutation at the position 9 is Q9I or Q9F.

In one or more embodiments, in the fusion protein, the polypeptide is selected from the group consisting of: SEQ ID NO: 2, 3, 6, 7, 8, 9, 11-19, 21-25, 58-62 and 65-69.

In one or more embodiments, linker sequence(s) is (are) present or absent between the polypeptides.

In one or more embodiments, the linker sequence(s) comprises (comprise) glycine (G) and serine (S).

In one or more embodiments, the C-terminal or N-terminal of the fusion protein further comprises one or more coupling elements.

In one or more embodiments, the coupling elements are selected from the group consisting of: cysteine residues, multiple lysine residues and multiple histidine residues.

In one or more embodiments, the fusion protein further includes residues from the excised signal transduction sequence.

In one or more embodiments, the amino acid sequence of the fusion protein is as shown in any one of SEQ ID NO: 75-108.

The fourth aspect of the present description provides a recombinant protein A, and the C domain of the recombinant protein A is the polypeptide described in any embodiment of the first aspect of the present description.

The fifth aspect of the present description provides a nucleic acid molecule, the polynucleotide sequence of the nucleic acid molecule is selected from:
(1) the polynucleotide sequence encoding the polypeptide described in any embodiment of the first aspect, the polypeptide described in any embodiment of the second aspect, the fusion protein described in any embodiment of the third aspect or the polynucleotide sequence of the recombinant protein A described in any embodiment of the fourth aspect of the present description;
(2) A polynucleotide sequence complementary to the polynucleotide sequence described in (1).

The sixth aspect of the present description provides a nucleic acid construct comprising the nucleic acid molecule described in the fifth aspect of the present description.

In one or more embodiments, the nucleic acid construct is an expression cassette.

In one or more embodiments, the nucleic acid construct is an expression vector or a cloning vector.

The seventh aspect of the present description also provides an expression system, which contains the nucleic acid construct or vector described in any embodiment of the sixth aspect of the present description.

In one or more embodiments, the expression system is a host cell.

The eighth aspect of the present description provides a separation medium, which comprises the polypeptide described in any embodiment of the first aspect of the present description, the polypeptide described in any embodiment of the second aspect, the fusion protein described in any embodiment of the third aspect and/or the recombinant protein A described in any embodiment of the fourth aspect, coupled to a solid support.

In one or more embodiments, the polypeptide, the fusion protein or the recombinant protein A is coupled to the solid support through a thioether bond.

The ninth aspect of the present description provides a chromatographic column, which contains the separation medium described in the eighth aspect of the present description.

The tenth aspect of the present description provides a method for isolating an Fc-containing protein (such as immunoglobulin), the method comprises a step where a sample containing the Fc-containing protein comes in contact with a polypeptide described in any embodiment of the first aspect, the polypeptide described in any embodiment of the second aspect, the fusion protein described in any embodiment of the third aspect and/or the recombinant protein A described in any embodiment of the fourth aspect of the present description.

In one or more embodiments, the method comprises the step where a sample containing the Fc-containing protein comes in contact with the separation medium described in any one embodiment of the eighth aspect or the chromatographic column described in the ninth aspect of the present description. Preferably, the method further comprises, washing the separation medium after the contacting, and eluting Fc-containing protein from the separation medium with an elution buffer.

The eleventh aspect of the present description provides the use of the polypeptide described in any embodiment of the first aspect, the polypeptide described in any embodiment of the second aspect, the fusion protein described in any embodiment of the third aspect and/or the recombinant protein A described in any embodiment of the fourth aspect of the present description, in separating Fc-containing proteins, or in the preparation of separation media or chromatographic columns for separating Fc-containing proteins.

### Description of Figures

Figure 1A: Gene result validation analysis diagram for SEQ ID NO: 74. 1: Plasmid; 2: Plasmid digested with XbaI-XhoI; 3: DNA Marker.
Figure 1B: Gene result validation analysis diagram for SEQ ID NO: 75.
Figure 1C: Gene result validation analysis diagram for SEQ ID NO: 76.
Figure 1D: Gene result validation analysis diagram for SEQ ID NO: 77.
Figure 1E: Gene result validation analysis diagram for SEQ ID NO: 78. 1: Plasmid; 2: Plasmid digested with XbaI-XhoI; 3: DNA Marker.
Figure 1F: Gene result validation analysis diagram for SEQ ID NO: 79. 1: Plasmid; 2: Plasmid digested with XbaI-XhoI; 3: DNA Marker.
Figure 1G: Gene result validation analysis diagram for SEQ ID NO: 80.
Figure 1H: Gene result validation analysis diagram for SEQ ID NO: 84. 1: Plasmid; 2: Plasmid digested with XbaI-XhoI; 3: DNA Marker.
Figure 1I: Gene result validation analysis diagram for SEQ ID NO: 85.
Figure 1J: Gene result validation analysis diagram for SEQ ID NO: 86.
Figure 1K: Gene result validation analysis diagram for SEQ ID NO: 87.
Figure 1L: Gene result validation analysis diagram for SEQ ID NO: 101. 1: Plasmid; 2: Plasmid digested with XbaI-XhoI; 3: DNA Marker.
Figure 1M: Gene result validation analysis diagram for SEQ ID NO: 103. The enzyme is Ndel/HindIII, and the expected size is 871+5k; the right panel is the DNA marker.
Figure 1N: Gene result validation analysis diagram for SEQ ID NO: 104. The enzyme is Ndel/HindIII, and the expected size is 871+5k; the right panel is the DNA marker.
Figure 2: Alkali-resistant stability curves of the C domains shown in SEQ ID NO: 74-90.
Figure 3: Alkali-resistant stability curves of the C domains shown in SEQ ID NO: 74, 92-94, 96-107.

### Detailed Description

It should be understood that within the scope of the present description, the above-mentioned technical features of the present description and the technical features specifically described in the following (such as the examples) can be combined with each other to form a preferred technical solution.

Herein, "antibody" and "immunoglobulin" are used interchangeably and have meanings well known in the art. Antibodies or immunoglobulins as described herein also comprise fragments of antibodies, and fusion proteins or conjugates containing fragments of antibodies, provided such Fc-containing antibody fragments, fusion proteins or conjugates can be separated and purified via binding with protein A. Fragments of an antibody may be functionally active fragments thereof.

In this specification, amino acid residues also use following abbreviations: alanine (Ala or A), arginine (Arg or R), asparagine (Asn or N), aspartate (Asp or D), Cysteine (Cys or C), Glutamine (Gln or Q), Glutamate (Glu or E), Glycine (Gly or G), Histidine (His or H), Isoleucine (Ile or 1), Leucine (Leu or L), Lysine (Lys or K), Methionine (Met or M), Phenylalanine (Phe or F), Proline (Pro or P), serine (Ser or S), threonine (Thr or T), tryptophan (Trp or W), tyrosine (Tyr or Y), valine (Val or V), and any amino acid residue (Xaa or X). In addition, in this specification, the amino acid sequence of a peptide is described so that the amino-terminus (hereinafter referred to as N-terminus) is on the left side and the carboxy-terminus (hereinafter referred to as C-terminus) is on the right side according to usual practice.

Protein A is widely used as an affinity medium ligand for the purification of immunoglobulins. Native protein A has five domains binding with immunoglobulins ( especially IgG), which are E domain, D domain, A domain, B domain and C domain from the N-terminus to C-terminus, respectively. In the process of immunoglobulin purification, the immunoglobulin needs to be eluted with alkali after combined with protein A. This process will cause protein A to shed from the affinity chromatography medium and cause contamination to the immunoglobulin product.

The present description finds that, when at least one of the positions 3, 6, 9 and 15 of the protein A native C domain undergoes substitution mutation (SEQ ID NO: 1), the resulted C domain mutant will witness significantly improved alkaline stability during alkaline cleaning process compared to its native counterparts. The concentration of alkali used can be increased from 0.1-0.5M to 0.5-2.0M(such as 0.5 - 1.0M). The present description is based on these facts.

Specifically, the present description provides a polypeptide, which is a mutant of the native C domain of the protein A shown in SEQ ID NO: 1. Compared with the amino acid sequence shown in SEQ ID NO: 1, the polypeptide has substitution mutations at one or more positions selected from the positions 3, 6, 9 and 15. The substitution mutation does not affect the original structure of the C domain, while significantly improves the alkaline stability of the obtained mutant.

Preferably, in the present description, the substitution at the position 3 is that native N is substituted by amino acid residues selected from glycine, alanine, valine, leucine, isoleucine, tyrosine or phenylalanine. Further preferably, the substitution at the position 3 is that native N is substituted by amino acid residues selected from leucine, valine or tyrosine. Particularly preferably, the substitution at the position 3 is that native N is substituted by leucine or valine. In some preferred embodiments, compared with SEQ ID NO: 1, the polypeptide of the present description has a substitution mutation at the position 3; preferably, the amino acid sequence of the polypeptide is shown in SEQ ID NO: 2, 3 or 4.

Preferably, in the present description, the substitution mutation at the position 6 is preferred to be that native N is substituted by glycine, alanine, serine, threonine, leucine, isoleucine, valine, glutamate or aspartate. Further preferably, the substitution at the position 6 is that native N is substituted by amino acid residues selected from serine, leucine or glutamate. Particularly preferably, the substitution at the position 6 is that native N is substituted by leucine or glutamate. In some preferred embodiments, compared with SEQ ID NO: 1, the polypeptide of the present description has a substitution mutation at the position 6; preferably, the amino acid sequence of the polypeptide is shown in SEQ ID NO: 5, 6 or 7.

Preferably, in the present description, the preferred substitution mutation at the position 9 is - native Q substituted by glycine, alanine, leucine, valine, tyrosine, serine, isoleucine, arginine, phenylalanine or methionine, more preferably by isoleucine, phenylalanine or methionine. In some preferred embodiments, compared with SEQ ID NO: 1, the polypeptide of the present description has a substitution mutation at the position 9; preferably, the amino acid sequence of the polypeptide is shown in SEQ ID NO: 8, 9 or 10.

Preferably, in the present description, the substitution mutation at the position 15 is that - native E is substituted by glycine, leucine, alanine, methionine, phenylalanine, tyrosine, isoleucine, serine, threonine, tryptophan, valine, aspartate, glutamate or arginine. Further preferably, the substitution at the position 15 is that the native E is substituted by amino acid residues selected from threonine, tryptophan, leucine, valine, isoleucine, phenylalanine, serine, tyrosine or aspartate. In some preferred embodiments, compared with SEQ ID NO: 1, the polypeptide of the present description has a substitution mutation at the position 15; preferably, the amino acid sequence of the polypeptide is shown in SEQ ID NO: 11-19.

In a preferred embodiment, compared with the native C domain of protein A shown in SEQ ID NO: 1, the polypeptide of the present description has substitution mutations at the following positions: Any two positions from position 3, 6, 9 and 15, any three positions from position 3, 6, 9 and 15 , or all four positions (3, 6, 9 and 15). Preferably, the substitution mutation is described as above. Further preferably, the substitution mutation at the position 9 is Q9I, Q9F or Q9M, the substitution mutation at the position 3 is N3L, N3V or N3Y, the substitution mutation at the position 6 is N6S, N6L or N6E, and the substitution mutation at the position 15 is E15T, E15W, E15L, E15V, E15I, E15F, E15S, E15Y or E15T.

In some embodiments, the polypeptide of the present description comprises the amino acid sequence shown in SEQ ID NO: 109: 
ADX1KFX2KEX3QNAFYX4ILHLPNLTEEQRNGFIQSLKDDPSVSKEILAEAKK
LNDAQAPK,
 wherein, X₁ is selected from glycine, alanine, valine, leucine, isoleucine, tyrosine or phenylalanine, preferably selected from leucine, valine or tyrosine; X₂ is selected from glycine, alanine, serine, threonine, leucine, isoleucine, valine, glutamate or aspartate, preferably selected from serine, leucine or glutamate; X₃ is selected from glycine, alanine, leucine, valine, tyrosine, serine, isoleucine, arginine, phenylalanine or methionine, preferably selected from isoleucine, phenylalanine or methionine; X₄ is selected from glycine, leucine, alanine, methionine, phenylalanine, tyrosine, isoleucine, serine, threonine, tryptophan, valine, aspartate, glutamate or arginine, preferably selected from threonine, tryptophan, leucine, valine, isoleucine, phenylalanine, serine, tyrosine or aspartate.

Further preferably, compared with the native C domain of the protein A shown in SEQ ID NO: 1, the polypeptide of the present description has a substitution mutation at the position 3, and optionally has a substitution mutation at one position selected from position 6, 9 and 15. Preferably, the substitution mutation is - described as - any of the embodiments mentioned above. Further preferably, the substitution mutation at the position 3 is N3L, N3V or N3Y; when the position 6 has a substitution mutation, the substitution mutation is N6S, N6L or N6E, preferably N6L or N6E; when the position 9 has a substitution mutation, the substitution mutation is Q9I or Q9F; when the position 15 has a substitution mutation, the substitution mutation is E15W, E15L, E15V, E15I or E15F.

Further preferably, compared with the native C domain of the protein A shown in SEQ ID NO: 1, the polypeptide of the present description has a substitution mutation at position 6, and optionally has a substitution mutation in one position selected from the position 3, 6 and 15. Preferably, the substitution mutation is - described as - any of the embodiments mentioned above. Further preferably, the substitution mutation at position 6 is N6S, N6L or N6E, preferably N6L or N6E; when the position 3 has a substitution mutation, the substitution mutation is N3L, N3V or N3Y; when the position 9 has a substitution mutation, the substitution mutation is Q9I or Q9F; when the position 15 has a substitution mutation, the substitution mutation is E15W, E15L, E15V, E15I or E15F.

Further preferably, compared with the native C domain of the protein A shown in SEQ ID NO: 1, the polypeptide of the present description has a substitution mutation at the position 9, and optionally has a substitution mutation in one position from position 3, 6 and 15. Preferably, the substitution mutation is described as above. Further preferably, the substitution mutation at position 9 is Q9I or Q9F, the substitution mutation at position 3 is N3L, N3V or N3Y, the substitution mutation at the position 6 is N6S, N6L or N6E, and the substitution mutation at the position 15 is E15T, E15W, E15L, E15V, E15I, E15F, E15S, E15Y or E15T. Further preferably, the substitution mutation at the position 9 is Q9I or Q9F; when the position 3 has a substitution mutation, the substitution mutation is N3L, N3V or N3Y; when the position 6 has a substitution mutation, the substitution mutation is N6S, N6L or N6E, preferably N6L or N6E; when the position 15 has a substitution mutation, the substitution mutation is E15W, E15L, E15V, E15I or E15F.

Further preferably, compared with the native C domain of the protein A shown in SEQ ID NO: 1, the polypeptide of the present description has a substitution mutation at the position 15, and optionally has a substitution mutation in one position selected from the position 3, 6 and 9. Preferably, the substitution mutation is described as above. Further preferably, the substitution mutation at the position 15 is E15T, E15W, E15L, E15V, E15I, E15F, E15S, E15Y or E15T, the substitution mutation at the position 9 is Q9I, Q9F or Q9M, the substitution mutation at the position 3 is N3L, N3V or N3Y, the substitution mutation at the position 6 is N6S, N6L or N6E. Further preferably, compared with the native C domain of the protein A shown in SEQ ID NO: 1, the polypeptide of the present description has a substitution mutation at the position 15, and optionally has a substitution mutation in one position selected from position 3, 6 and 9; preferably, the exemplary amino acid sequence of the polypeptide is shown in SEQ ID NO: 20-37. Further preferably, compared with the native C domain of the protein A shown in SEQ ID NO: 1, the polypeptide of the present description has a substitution mutation at the position 15, and optionally has substitution mutation in two positions selected from the positions 3, 6 and 9; preferably, the exemplary amino acid sequence of the polypeptide is shown in SEQ ID NO: 38-57. Further preferably, compared with the native C domain of the protein A shown in SEQ ID NO: 1, the polypeptide of the present description has a substitution mutation at the position 15, as well as substitution mutations at position 3, 6 and 9; preferably, the exemplary amino acid sequence of the polypeptide is shown in SEQ ID NO: 58-73.

In some preferred embodiments, compared with the native C domain of the protein A shown in SEQ ID NO: 1, the polypeptide of the present description has substitution mutations at position 9 and 15, and optionally has substitution mutations at one or two positions selected from position 3 and 6. In a further preferable embodiment, compared with the native C domain of the protein A shown in SEQ ID NO: 1, the polypeptide has substitution mutations at position 3, 9 and 15, and optionally has substitution mutation at position 6. Preferably, the substitution mutation is described as above. Further preferably, the substitution mutation at position 9 is Q9I, Q9F or Q9M, the substitution mutation at position 3 is N3L, N3V or N3Y, the substitution mutation at position 6 is N6S, N6L or N6E, and the substitution mutation at position 15 is E15T, E15W, E15L, E15V, E151, E15F, E15S, E15Y or E15T.

In some preferred embodiments, compared with the native C domain of the protein A shown in SEQ ID NO: 1, the polypeptide of the present description has substitution mutations at position 3, 6, 9 and 15; wherein, the substitution mutation at position 3 is N3L or N3V, substitution mutation at position 6 is N6L or N6E, substitution mutation at position 9 is Q9I or Q9F, and substitution mutation at position 15 is E15W, E15V, E15I or E15L.

It is acknowledged in the field that by mutating the amino acid residue G at position 29 of the protein A native C domain into A, alkali resistance of the resulting mutant can be improved as a result. Therefore, in a preferred embodiment of the present description, in addition to the mutation at one or more positions from position 3, 6, 9 and 15, the polypeptide of the present description may also has a G29A mutation at position 29.

The polypeptide of the present description can be directly used as a ligand for binding and separating immunoglobulins. Accordingly, in some embodiments, the polypeptide further comprises one or more coupling elements at its C- or N-terminus for coupling the polypeptide to a solid support. Suitable coupling elements are well known in the art , including but not limited to, cysteine residues, multiple lysine residues (such as 3-15 or 5-10) and multiple histidine residues (such as 3-15 or 5-10). The coupled element can be a cysteine residue located at the C-terminal of the polypeptide, allowing the polypeptide to be coupled to the solid support by reaction between the thiol group in the cysteine residue and the electrophilic group on the solid support. A coupled element can be directly connected to the polypeptide, alternatively, it can be connected to the N- or C-terminus of the polypeptide via a linker. The linker may be a linker commonly used in the art, and its presence will not affect the binding activity of the polypeptide. An exemplary linker is a linker sequence having or consisting of G and S, typically 2-20 amino acid residues in length. Exemplary linker sequences such as (GS)ₙ, (GSS)ₙ and (GSSS)ₙ, among which n can be an integer ranging from 2 to 10, while the total length of the linker will normally not exceed 20 amino acid residues. The polypeptide may also comprise multiple amino acid residues (e.g., fewer than 15, 10 or 5) at the N-terminus, which are derived from the cloning process or residues from signal transduction sequences. For example, the polypeptide may comprise AQ at the N-terminus.

The present description also provides a fusion protein, which is formed by the fusion of 2-8 aforementioned polypeptides of the present description. The polypeptides forming the fusion protein may be all different, partly or entirely identical. Each polypeptide can be directly connected through the peptide bonds at the C-terminal and N-terminal, respectively. Alternatively, any two polypeptides can also be connected through a linker. The linker may be a linker commonly used in the art, and its presence will not affect the binding activity of the polypeptide. An exemplary linker is a linker sequence comprising or consisting of G and S, typically 2-20 amino acid residues in length. Exemplary linker sequences such as (GS)ₙ, (GSS)ₙ and (GSSS)ₙ, among which n can be an integer ranging from 2 to 10, while the total length of the linker will normally not exceed 20 amino acid residues.

The fusion protein can be used as a ligand for binding and separating immunoglobulins. Accordingly, in some embodiments, the fusion protein further comprises one or more coupling elements at its C- or N-terminus for coupling the fusion protein to a solid support. Suitable coupling elements are well known in the art including but not limited to, cysteine residues, multiple lysine residues (such as 3-15 or 5-10) and multiple histidine residues (such as 3-15 or 5-10). The coupled element can be a cysteine residue located at the C-terminal of the fusion protein, allowing the fusion protein to be coupled to the solid support by reaction between the thiol group in the cysteine residue and the electrophilic group ( 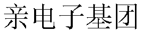 ) on the solid support. The coupled element can be directly connected to the polypeptide, alternatively, it can be connected to the N- or C-terminus of the polypeptide via a linker. The linker may be a linker commonly used in the art, and its presence will not affect the binding activity of the polypeptide. An exemplary linker is a linker sequence comprising or consisting of G and S, typically 2-20 amino acid residues in length. Exemplary linker sequences such as (GS)ₙ, (GSS)ₙ and (GSSS)ₙ, among which n can be an integer ranging from 2 to 10,while the total length of the linker will normally not exceed 20 amino acid residues. The fusion protein may also comprise multiple amino acid residues (e.g., fewer than 15, 10 or 5) at the N-terminus, derived from the cloning process or residues from signal transduction sequences. As a specific example, the fusion protein may comprise AQ at its N-terminus. The amino acid sequence of an exemplary fusion protein is shown in any one of SEQ ID NO: 75-108.

The present description also provides a recombinant protein A, whose C domain is the polypeptide described in any embodiment of the present description. As it is well-known in the field, the rest of the domains of the recombinant protein A can be the E domain, D domain, A domain and B domain (or Z domain, which is a remodeled domain of the B domain), including native E domain, D domain, A domain and B domain as well as known mutations but retaining biological activity of E domain, D domain, A domain and/or B domain (such as Z domain). Except for the C domain using the polypeptide of the present description, the known mutations or modifications to the rest domains of the protein A can all be used in the recombinant protein A of the present description.

This present description also includes peptides, fusion proteins, or recombinant protein A having sequence having at least 85% identity compared with the peptides, fusion proteins, or recombinant protein A of any of the embodiments described in the present description, and having substitution mutations as described in any one of the embodiments at one or more amino acid positions corresponding to position 3, 6, 9 and 15 of SEQ ID NO: 1; preferably, the amino acid residue corresponding to the position 29 of the protein A native domain C in such peptide is A, as well as that in such peptide of the fusion protein and the recombinant protein A. Herein, the "at least 85% identity" related to the amino acid sequence and the nucleotide sequence refers to an identity of more than 85%, preferably more than 90%, more preferably more than 95%, and even more preferably more than 97%, more preferably more than 98%, and still more preferably more than 99%. The identity between two aligned sequences can be calculated using tools well known in the art, including BLASTP for amino acid sequence alignment and so on. In preferred embodiments, the present description comprises mutants of any amino acid sequence shown in SEQ ID NO: 2-73 and 75-108, each with at least 85%, preferably at least 90%, more preferably at least 97%, more preferably at least 99% sequence similarity compared to the corresponding parent sequence, and their amino acid residues at the positions 3, 6, 9 and 15 of the corresponding parent sequences remain the same as the corresponding parent amino acid residues.

The present description also includes the nucleic acid molecules encoding the aforementioned polypeptides, fusion proteins and recombinant protein A and their complementary sequences. The description includes all forms of the nucleic acid molecules described in the description, including RNA and DNA. It should be understood that the complementary sequence described herein refers to a complementary sequence whose length is substantially the same as that of the nucleic acid molecule.

Nucleic acid constructs comprising the nucleic acid molecule or its complement are also covered by the scope of the present description. The nucleic acid construct may be an expression cassette comprising a promoter, the nucleic acid molecule and a transcription termination sequence. Among which, promoters and transcription termination sequences are well known in the art, and can be appropriately selected by those skilled in the art according to the host cell selected for expressing the polypeptide, fusion protein or recombinant protein A.

In some embodiments, nucleic acid constructs are vectors, including expression vectors and cloning vectors. Expression vectors are suitable for expressing exogenous genes in host cells, for example, nucleic acid molecules encoding polypeptides, fusion protein or recombinant protein A described in this description, including prokaryotic expression vectors and eukaryotic expression vectors. Eukaryotic expression systems include yeast expression systems, mammalian cell expression systems and insect cell expression systems. The cloning vector is used to amplify the target gene ( e.g. the nucleic acid molecule encoding the polypeptide, fusion protein or protein A of the present description) in the host cell. Cloning vectors include plasmid vectors, phage vectors, viral vectors, as well as vectors combined with each other or another genomic DNA. The most commonly used host cell in molecular cloning is *Escherichia coli.*

The present description also provides an expression system comprising the nucleic acid molecule, nucleic acid construct or vector disclosed herein. The expression system can be, for example, a Gram-positive or Gram-negative prokaryotic host cell system, such as E.coli or Bacillus that has been modified to express the peptides, the fusion proteins, or the recombinant protein A of the present description. In some embodiments, the expression system is a eukaryotic host cell system, such as yeast, such as *Pichia pastoris* or *Saccharomyces cerevisiae.*

The present description also provides a separation medium. The separation medium of the present description can be used to separate immunoglobulins or Fc-containing proteins. The separation medium of the present description contains the polypeptide, fusion protein and/or recombinant protein A described in any embodiment of the present description coupled to a solid support. Due to the improved alkaline stability of the polypeptide, fusion protein and recombinant protein A concerned in the present description, the separation medium of the present description can withstand highly alkaline conditions (0.5-2.0M NaOH) during cleaning.

The solid support (matrix) of separation medium in the present description may be a solid support (matrix) known in the art which is used in the separation of immunoglobulin or Fc-containing protein.

Herein, solid phase support may be in any shape including particle, film, plate, tube, needle, and fiber. The solid support of the present description may be porous or non-porous material. In some embodiments, the solid support is in the form of porous or non-porous beads or particles. Matrix in beaded or granular form can be used as packed beds or suspension. Suspension includes expanded beds in which the particles or beads can move freely.

When the solid phase support is a particle, its particle size is preferably 20-200 µm. For example, in case of solid support is a synthetic polymer, the particle size is preferably 20-100 µm, more preferably 30-80 µm. When the solid phase support is polysaccharide, the particle size is preferably 50-200 µm, more preferably 60-150 µm. The "particle size" in this specification refers to the average volume particle size measured by the ektacytometry based on ISO 13320 and JIS Z 8825-1.

Examples of solid supports include, but are not limited to, polymers containing polyhydroxyl groups, such as polysaccharides. Polysaccharides include dextran, starch, cellulose, pullulan, agar and agarose, etc. In preferred embodiments, the solid support comprises agar or agarose.

In some embodiments, the solid supports of the description are synthetic polymers with hydrophilic surfaces. For example, such polymers can be synthetic polymers with hydroxyl, carboxyl, amino carbonyl, amino or oligoethylene oxide or polyethylene oxide groups on the outer surface (and on the inner surface if presence) through hydrophilization treatment, preferably synthetic polymer obtained by crosslinking multifunctional monomers such as methyl methacrylate and divinylbenzene. Exemplary synthetic polymers include polyvinyl alcohol, polystyrene, polystyrene divinylbenzene, polyhydroxyalkyl acrylates, polyhydroxyalkyl methacrylates, polyacrylamides, polymethacrylamides, and the like. In the case of polymer whose surface is hydrophobic, the surface can be hydrophilized to expose the hydrophilic groups to the surrounding aqueous liquid.

In some embodiments, the solid supports of the present description comprise supports of inorganic nature, including but not limited to, silica, zirconia, and the like.

In some embodiments, the solid support of the description is in the form of a surface, chip, capillary or filter, e.g., a membrane or the like.

In the present description, the polypeptide, fusion protein and recombinant protein A of the present description can be connected to the corresponding solid support through conventional coupling techniques through the thiol group, amino group and/or carboxyl group contained therein. Commonly used coupling reagents include, but are not limited to, diepoxides, epichlorohydrin, CNBr, and N-hydroxysuccinimide. A spacer can be introduced between the solid support and the polypeptide, fusion protein and recombinant protein A of the present description, which can facilitate their chemical coupling with the solid support.

In some embodiments, solid support (matrix) can be in the forms of surface, chip, capillary or filter, for example, membrane and so on.

In the present description, polypeptide, fusion protein or recombinant protein A can be coupled to solid support via Mercaptan group, amino group and/or carboxyl group by normal coupling technology. Commonly used coupling reagents include but not limited to bicyclic oxide, epichlorol, CNBr and hydroxysuccinimide. Besides, spacer arms can be introduced between solid support and polypeptide, fusion protein or recombinant protein A to promote the chemical coupling between them.

In some embodiments, the polypeptide, fusion protein or recombinant protein A is coupled to the solid support through a thioether bond. In some embodiments, the polypeptide, fusion protein or recombinant protein A of the present description is coupled through its C-terminal cysteine, wherein the cysteine thiol group is effectively coupled to the electrophilic group (such as epoxy group, halohydrin group, etc.) on the solid support, forming a thioether bridge coupling.

In the separation medium of the present description, the concentration of the ligands coupled to the solid support (such as polypeptide, fusion protein and/or recombinant protein A as described in the present description) is usually 5-20 mg/ml (e.g. 5-15 mg/ml). The amount of ligands coupled can be controlled by adjusting the concentration of the polypeptide, fusion protein and/or recombinant protein A used in the coupling process, the coupling conditions and/or the pore structure of the solid support.

The present description also provides a chromatographic column containing the separation medium of the present description. Usually, the separation medium the present description is packed in the chromatographic column.

The present description also provides a method for separating Fc-containing protein (such as immunoglobulin), the method comprising the steps where peptide, the fusion protein, and/or the recombinant protein A described in any embodiment of the present description is brought into contact with Fc-containing protein samples. In some embodiments, the method includes the step where samples are in contact with the separation medium or chromatography column described in any of the embodiments herein.

More specifically, in some embodiments, the method includes:
(1) bringing Fc-containing protein sample into contact with the separation medium according to any of the embodiments herein;
(2) washing the separation medium;
(3) eluting the Fc-containing protein from the separation medium;
(4) washing the separation medium;

Herein, the samples may be various types of Fc-containing proteins (especially immunoglobulins). Preferably, the immunoglobulin described herein refers to IgG.

The washing buffer used in the method for washing the separation medium, the elution buffer for eluting the protein and the cleaning buffer for cleaning the separation medium are all commonly used washing buffers, elution buffers, and cleaning buffers in this field (especially in the field of protein A chromatography). For example, the washing buffer can be PBS solution. The elution buffer can be a solution or buffer with (pH≤5), preferably, the pH of the elution buffer is 2.5-5 or 3-5. In some embodiments, the pH of the elution buffer is 11 or higher, e.g., the pH of the elution buffer is 11-14 or 11-13. In some embodiments, elution is performed using a citrate solution (e.g., a sodium citrate solution). Cleaning buffers are usually alkaline and can have a pH of 13-14. In some embodiments, the cleaning buffer is sodium hydroxide solution or potassium hydroxide solution with concentration of 0.1-2.0 M (e.g., 0.5-2.0 M or 0.5-1.0 M).

In some embodiments, the methods described herein further includes steps of collecting the eluate, and further separating and purifying the eluate. According to the separated protein, further separation and purification can be carried out by anion or cation exchange chromatography, mixed-mode chromatography and/or hydrophobic interaction chromatography, etc.

The present description also provides the use of the polypeptide, fusion protein and protein A described in any embodiment herein in the separation and purification process of Fc-containing proteins, or in the preparation for the separation medium and purification of Fc-containing proteins.

The sequence described in the present description is as follows:
SEQ ID NO: 1 (Native C domain) 
ADNKFNKEQQNAFYEILHLPNLTEEQRNGFIQSLKDDPSVSKEILAEAK
KLNDAQAPK
SEQ ID NO: 2 (N3L) 
ADLKFNKEQQNAFYEILHLPNLTEEQRNGFIQSLKDDPSVSKEILAEAK
KLNDAQAPK
 SEQ ID NO: 3 (N3V) 
ADVKFNKEQQNAFYEILHLPNLTEEQRNGFIQSLKDDPSVSKEILAEAK
KLNDAQAPK
SEQ ID NO: 4 (N3Y) ADYKFNKEQQNAFYEILHLPNLTEEQRNGFIQSLKDDPSVSKEILAEAK KLNDAQAPK
SEQ ID NO: 5 (N6S) 
ADNKFSKEQQNAFYEILHLPNLTEEQRNGFIQSLKDDPSVSKEILAEAK
KLNDAQAPK
SEQ ID NO: 6 (N6L) 
ADNKFLKEQQNAFYEILHLPNLTEEQRNGFIQSLKDDPSVSKEILAEAK
KLNDAQAPK
SEQ ID NO: 7 (N6E) 
ADNKFEKEQQNAFYEILHLPNLTEEQRNGFIQSLKDDPSVSKEILAEAK
KLNDAQAPK
SEQ ID NO: 8 (Q9I) 
ADNKFNKEIQNAFYEILHLPNLTEEQRNGFIQSLKDDPSVSKEILAEAK
KLNDAQAPK
SEQ ID NO: 9 (Q9F) 
ADNKFNKEFQNAFYEILHLPNLTEEQRNGFIQSLKDDPSVSKEILAEAK
KLNDAQAPK
SEQ ID NO: 10 (Q9M) 
ADNKFNKEMQNAFYEILHLPNLTEEQRNGFIQSLKDDPSVSKEILAEAK
KLNDAQAPK
SEQ ID NO: 11 (E15T) 
ADNKFNKEQQNAFYTILHLPNLTEEQRNGFIQSLKDDPSVSKEILAEAK
KLNDAQAPK
SEQ ID NO: 12 (E15W) 
ADNKFNKEQQNAFYWILHLPNLTEEQRNGFIQSLKDDPSVSKEILAEA
KKLNDAQAPK
SEQ ID NO: 13 (E15L) 
ADNKFNKEQQNAFYLILHLPNLTEEQRNGFIQSLKDDPSVSKEILAEAK
KLNDAQAPK
SEQ ID NO: 14 (E15V) 
ADNKFNKEQQNAFYVILHLPNLTEEQRNGFIQSLKDDPSVSKEILAEAK
KLNDAQAPK
SEQ ID NO: 15 (E15I) 
ADNKFNKEQQNAFYIILHLPNLTEEQRNGFIQSLKDDPSVSKEILAEAK
KLNDAQAPK
SEQ ID NO: 16 (E15F) 
ADNKFNKEQQNAFYFILHLPNLTEEQRNGFIQSLKDDPSVSKEILAEAK
KLNDAQAPK
SEQIDNO: 17(E15S) 
ADNKFNKEQQNAFYSILHLPNLTEEQRNGFIQSLKDDPSVSKEILAEAK
KLNDAQAPK
SEQ ID NO: 18 (E15Y) 
ADNKFNKEQQNAFYYILHLPNLTEEQRNGFIQSLKDDPSVSKEILAEAK
KLNDAQAPK
SEQ ID NO: 19 (E15D) 
ADNKFNKEQQNAFYDILHLPNLTEEQRNGFIQSLKDDPSVSKEILAEAK
KLNDAQAPK
SEQ ID NO: 20 (Q9I,E15T) 
ADNKFNKEIQNAFYTILHLPNLTEEQRNGFIQSLKDDPSVSKEILAEAK
KLNDAQAPK
SEQ ID NO: 21 (Q9I,E15W) 
ADNKFNKEIQNAFYWILHLPNLTEEQRNGFIQSLKDDPSVSKEILAEAK
KLNDAQAPK
SEQ ID NO: 22 (Q9I,E15L) 
ADNKFNKEIQNAFYLILHLPNLTEEQRNGFIQSLKDDPSVSKEILAEAK
KLNDAQAPK
SEQ ID NO: 23 (Q9I,E15V) 
ADNKFNKEIQNAFYVILHLPNLTEEQRNGFIQSLKDDPSVSKEILAEAK
KLNDAQAPK
SEQ ID NO: 24 (Q9I,E15I) 
ADNKFNKEIQNAFYIILHLPNLTEEQRNGFIQSLKDDPSVSKEILAEAKK
LNDAQAPK
SEQ ID NO: 25 (Q9I,E15F) 
ADNKFNKEIQNAFYFILHLPNLTEEQRNGFIQSLKDDPSVSKEILAEAKK
LNDAQAPK
SEQ ID NO: 26 (Q9I,E15S) 
ADNKFNKEIQNAFYSILHLPNLTEEQRNGFIQSLKDDPSVSKEILAEAKK
LNDAQAPK
SEQ ID NO: 27 (Q9I,E15Y) 
ADNKFNKEIQNAFYYILHLPNLTEEQRNGFIQSLKDDPSVSKEILAEAK
KLNDAQAPK
SEQ ID NO: 28 (Q9I,E15D) 
ADNKFNKEIQNAFYDILHLPNLTEEQRNGFIQSLKDDPSVSKEILAEAK
KLNDAQAPK
SEQ ID NO: 29 (Q9F,E15T) 
ADNKFNKEFQNAFYTILHLPNLTEEQRNGFIQSLKDDPSVSKEILAEAK
KLNDAQAPK
SEQ ID NO: 30 (Q9F,E15W) 
ADNKFNKEFQNAFYWILHLPNLTEEQRNGFIQSLKDDPSVSKEILAEAK
KLNDAQAPK
SEQ ID NO: 31 (Q9F,E15L) 
ADNKFNKEFQNAFYLILHLPNLTEEQRNGFIQSLKDDPSVSKEILAEAK
KLNDAQAPK
SEQ ID NO: 32 (Q9F,E15V) ADNKFNKEFQNAFYVILHLPNLTEEQRNGFIQSLKDDPSVSKEILAEAK KLNDAQAPK
SEQ ID NO: 33 (Q9F,E15I) 
ADNKFNKEFQNAFYIILHLPNLTEEQRNGFIQSLKDDPSVSKEILAEAKK
LNDAQAPK
SEQ ID NO: 34 (Q9F,E15F) 
ADNKFNKEFQNAFYFILHLPNLTEEQRNGFIQSLKDDPSVSKEILAEAK
KLNDAQAPK
SEQ ID NO: 35 (Q9F,E15S) 
ADNKFNKEFQNAFYSILHLPNLTEEQRNGFIQSLKDDPSVSKEILAEAK
KLNDAQAPK
SEQ ID NO: 36 (Q9F,E15Y) 
ADNKFNKEFQNAFYYILHLPNLTEEQRNGFIQSLKDDPSVSKEILAEAK
KLNDAQAPK
SEQ ID NO: 37 (Q9F,E15D) 
ADNKFNKEFQNAFYDILHLPNLTEEQRNGFIQSLKDDPSVSKEILAEAK
KLNDAQAPK
SEQ ID NO: 38 (N3L,Q9I,E15W) 
ADLKFNKEIQNAFYWILHLPNLTEEQRNGFIQSLKDDPSVSKEILAEAK
KLNDAQAPK
SEQ ID NO: 39 (N3L,Q9I,E15L) 
ADLKFNKEIQNAFYLILHLPNLTEEQRNGFIQSLKDDPSVSKEILAEAKK
LNDAQAPK
SEQ ID NO: 40 (N3L,Q9I,E15V) 
ADLKFNKEIQNAFYVILHLPNLTEEQRNGFIQSLKDDPSVSKEILAEAK
KLNDAQAPK
SEQ ID NO: 41 (N3L,Q9I,E15I) 
ADLKFNKEIQNAFYIILHLPNLTEEQRNGFIQSLKDDPSVSKEILAEAKK
LNDAQAPK
SEQ ID NO: 42 (N3L,Q9I,E15F) 
ADLKFNKEIQNAFYFILHLPNLTEEQRNGFIQSLKDDPSVSKEILAEAKK
LNDAQAPK
SEQ ID NO: 43 (N3V,Q9I,E15W) 
ADVKFNKEIQNAFYWILHLPNLTEEQRNGFIQSLKDDPSVSKEILAEAK
KLNDAQAPK
SEQ ID NO: 44 (N3V,Q9I,E15L) 
ADVKFNKEIQNAFYLILHLPNLTEEQRNGFIQSLKDDPSVSKEILAEAK
KLNDAQAPK
SEQ ID NO: 45 (N3V,Q9I,E15V) 
ADVKFNKEIQNAFYVILHLPNLTEEQRNGFIQSLKDDPSVSKEILAEAK
KLNDAQAPK
SEQ ID NO: 46 (N3V,Q9I,E15I) 
ADVKFNKEIQNAFYIILHLPNLTEEQRNGFIQSLKDDPSVSKEILAEAKK
LNDAQAPK
SEQ ID NO: 47 (N3V,Q9I,E15F) 
ADVKFNKEIQNAFYFILHLPNLTEEQRNGFIQSLKDDPSVSKEILAEAKK
LNDAQAPK
SEQ ID NO: 48 (N6L,Q9F,E15W) 
ADNKFLKEFQNAFYWILHLPNLTEEQRNGFIQSLKDDPSVSKEILAEAK
KLNDAQAPK
SEQ ID NO: 49 (N6L,Q9F,E15L) 
ADNKFLKEFQNAFYLILHLPNLTEEQRNGFIQSLKDDPSVSKEILAEAK
KLNDAQAPK
SEQ ID NO: 50 (N6L,Q9F,E15V) 
ADNKFLKEFQNAFYVILHLPNLTEEQRNGFIQSLKDDPSVSKEILAEAK
KLNDAQAPK
SEQ ID NO: 51 (N6L, Q9F,E15I) 
ADNKFLKEFQNAFYIILHLPNLTEEQRNGFIQSLKDDPSVSKEILAEAKK
LNDAQAPK
SEQ ID NO: 52 (N6L,Q9F,E15F) 
ADNKFLKEFQNAFYFILHLPNLTEEQRNGFIQSLKDDPSVSKEILAEAK
KLNDAQAPK
SEQ ID NO: 53 (N6E,Q9F,E15W) 
ADNKFEKEFQNAFYWILHLPNLTEEQRNGFIQSLKDDPSVSKEILAEAK
KLNDAQAPK
SEQ ID NO: 54 (N6E,Q9F,E15L) 
ADNKFEKEFQNAFYLILHLPNLTEEQRNGFIQSLKDDPSVSKEILAEAK
KLNDAQAPK
SEQ ID NO: 55 (N6E,Q9F,E15V) 
ADNKFEKEFQNAFYVILHLPNLTEEQRNGFIQSLKDDPSVSKEILAEAK
KLNDAQAPK
SEQ ID NO: 56 (N6E, Q9F,E15I) 
ADNKFEKEFQNAFYIILHLPNLTEEQRNGFIQSLKDDPSVSKEILAEAKK
LNDAQAPK
SEQ ID NO: 57 (N6E,Q9F,E15F) 
ADNKFEKEFQNAFYFILHLPNLTEEQRNGFIQSLKDDPSVSKEILAEAK
KLNDAQAPK
SEQ ID NO: 58 (N3L,N6L,Q9I,E15W) 
ADLKFLKEIQNAFYWILHLPNLTEEQRNGFIQSLKDDPSVSKEILAEAK
KLNDAQAPK
SEQ ID NO: 59 (N3L,N6L,Q9I,E15L) 
ADLKFLKEIQNAFYLILHLPNLTEEQRNGFIQSLKDDPSVSKEILAEAKK
LNDAQAPK
SEQ ID NO: 60 (N3Y,N6E,Q9I,E15L) ADYKFEKEIQNAFYLILHLPNLTEEQRNGFIQSLKDDPSVSKEILAEAKK LNDAQAPK
SEQ ID NO: 61 (N3Y,N6E,Q9I,E15I) 
ADYKFEKEIQNAFYIILHLPNLTEEQRNGFIQSLKDDPSVSKEILAEAKK
LNDAQAPK
SEQ ID NO: 62 (N3V,N6L,Q9I,E15V) 
ADVKFLKEIQNAFYVILHLPNLTEEQRNGFIQSLKDDPSVSKEILAEAK
KLNDAQAPK
SEQ ID NO: 63 (N3V,N6E,Q9I,E15L) 
ADVKFEKEIQNAFYLILHLPNLTEEQRNGFIQSLKDDPSVSKEILAEAKK
LNDAQAPK
SEQ ID NO: 64 (N3V,N6E,Q9I,E15I) 
ADVKFEKEIQNAFYIILHLPNLTEEQRNGFIQSLKDDPSVSKEILAEAKK
LNDAQAPK
SEQ ID NO: 65 (N3V,N6E,Q9I,E15F) 
ADVKFEKEIQNAFYFILHLPNLTEEQRNGFIQSLKDDPSVSKEILAEAKK
LNDAQAPK
SEQ ID NO: 66 (N3L,N6L,Q9F,E15W) 
ADLKFLKEFQNAFYWILHLPNLTEEQRNGFIQSLKDDPSVSKEILAEAK
KLNDAQAPK
SEQ ID NO: 67 (N3L,N6L,Q9F,E15I) 
ADLKFLKEFQNAFYIILHLPNLTEEQRNGFIQSLKDDPSVSKEILAEAKK
LNDAQAPK
SEQ ID NO: 68 (N3L,N6E,Q9F,E15L) 
ADLKFEKEFQNAFYLILHLPNLTEEQRNGFIQSLKDDPSVSKEILAEAK
KLNDAQAPK
SEQ ID NO: 69 (N3V,N6L,Q9F,E15V) 
ADVKFLKEFQNAFYVILHLPNLTEEQRNGFIQSLKDDPSVSKEILAEAK
KLNDAQAPK
SEQ ID NO: 70 (N3V,N6E,Q9F,E15I) 
ADVKFEKEFQNAFYIILHLPNLTEEQRNGFIQSLKDDPSVSKEILAEAKK
LNDAQAPK
SEQ ID NO: 71 (N3V,N6E,Q9F,E15F) 
ADVKFEKEFQNAFYFILHLPNLTEEQRNGFIQSLKDDPSVSKEILAEAK
KLNDAQAPK
SEQ ID NO: 72 (N3L,N6E,Q9F,E15I) 
ADLKFEKEFQNAFYIILHLPNLTEEQRNGFIQSLKDDPSVSKEILAEAKK
LNDAQAPK
SEQ ID NO: 73 (N3V,N6L,Q9F,E15L) 
ADVKFLKEFQNAFYLILHLPNLTEEQRNGFIQSLKDDPSVSKEILAEAK
KLNDAQAPK
SEQ ID NO: 74 (Native C domain) 4 
ADNKFNKEQQNAFYEILHLPNLTEEQRNGFIQSLKDDPSVSKEILAEAK
KLNDAQAPKADNKFNKEQQNAFYEILHLPNLTEEQRNGFIQSLKDDPSVSK
EILAEAKKLNDAQAPKADNKFNKEQQNAFYEILHLPNLTEEQRNGFIQSLK
DDPSVSKEILAEAKKLNDAQAPKADNKFNKEQQNAFYEILHLPNLTEEQRN
GFIQSLKDDPSVSKEILAEAKKLNDAQAPK
SEQ ID NO: 75 (N3L)4 
ADLKFNKEQQNAFYEILHLPNLTEEQRNGFIQSLKDDPSVSKEILAEAK
KLNDAQAPKADLKFNKEQQNAFYEILHLPNLTEEQRNGFIQSLKDDPSVSK
EILAEAKKLNDAQAPKADLKFNKEQQNAFYEILHLPNLTEEQRNGFIQSLKD
DPSVSKEILAEAKKLNDAQAPKADLKFNKEQQNAFYEILHLPNLTEEQRNG
FIQSLKDDPSVSKEILAEAKKLNDAQAPK
SEQ ID NO: 76 (N3V)4 
ADVKFNKEQQNAFYEILHLPNLTEEQRNGFIQSLKDDPSVSKEILAEAK
KLNDAQAPKADVKFNKEQQNAFYEILHLPNLTEEQRNGFIQSLKDDPSVSK
EILAEAKKLNDAQAPKADVKFNKEQQNAFYEILHLPNLTEEQRNGFIQSLK
 
DDPSVSKEILAEAKKLNDAQAPKADVKFNKEQQNAFYEILHLPNLTEEQRN
GFIQSLKDDPSVSKEILAEAKKLNDAQAPK
SEQ ID NO: 77 (N6L)4 
ADNKFLKEQQNAFYEILHLPNLTEEQRNGFIQSLKDDPSVSKEILAEAK
KLNDAQAPKADNKFLKEQQNAFYEILHLPNLTEEQRNGFIQSLKDDPSVSK
EILAEAKKLNDAQAPKADNKFLKEQQNAFYEILHLPNLTEEQRNGFIQSLKD
DPSVSKEILAEAKKLNDAQAPKADNKFLKEQQNAFYEILHLPNLTEEQRNG
FIQSLKDDPSVSKEILAEAKKLNDAQAPK
SEQ ID NO: 78 (N6E)4 
ADNKFEKEQQNAFYEILHLPNLTEEQRNGFIQSLKDDPSVSKEILAEAK
KLNDAQAPKADNKFEKEQQNAFYEILHLPNLTEEQRNGFIQSLKDDPSVSK
EILAEAKKLNDAQAPKADNKFEKEQQNAFYEILHLPNLTEEQRNGFIQSLKD
DPSVSKEILAEAKKLNDAQAPKADNKFEKEQQNAFYEILHLPNLTEEQRNG
FIQSLKDDPSVSKEILAEAKKLNDAQAPK
SEQ ID NO: 79 (Q9I)4 
ADNKFNKEIQNAFYEILHLPNLTEEQRNGFIQSLKDDPSVSKEILAEAK
KLNDAQAPKADNKFNKEIQNAFYEILHLPNLTEEQRNGFIQSLKDDPSVSKE
ILAEAKKLNDAQAPKADNKFNKEIQNAFYEILHLPNLTEEQRNGFIQSLKDD
PSVSKEILAEAKKLNDAQAPKADNKFNKEIQNAFYEILHLPNLTEEQRNGFI
QSLKDDPSVSKEILAEAKKLNDAQAPK
SEQ ID NO: 80 (Q9F)4 
ADNKFNKEFQNAFYEILHLPNLTEEQRNGFIQSLKDDPSVSKEILAEAK
KLNDAQAPKADNKFNKEFQNAFYEILHLPNLTEEQRNGFIQSLKDDPSVSK
EILAEAKKLNDAQAPKADNKFNKEFQNAFYEILHLPNLTEEQRNGFIQSLKD
DPSVSKEILAEAKKLNDAQAPKADNKFNKEFQNAFYEILHLPNLTEEQRNG
FIQSLKDDPSVSKEILAEAKKLNDAQAPK
SEQ ID NO: 81 (Q9M)4 ADNKFNKEMQNAFYEILHLPNLTEEQRNGFIQSLKDDPSVSKEILAEAK 
KLNDAQAPKADNKFNKEMQNAFYEILHLPNLTEEQRNGFIQSLKDDPSVSK
EILAEAKKLNDAQAPKADNKFNKEMQNAFYEILHLPNLTEEQRNGFIQSLK
DDPSVSKEILAEAKKLNDAQAPKADNKFNKEMQNAFYEILHLPNLTEEQRN
GFIQSLKDDPSVSKEILAEAKKLNDAQAPK
SEQ ID NO: 82 (E15T)4 
ADNKFNKEQQNAFYTILHLPNLTEEQRNGFIQSLKDDPSVSKEILAEAK
KLNDAQAPKADNKFNKEQQNAFYTILHLPNLTEEQRNGFIQSLKDDPSVSK
EILAEAKKLNDAQAPKADNKFNKEQQNAFYTILHLPNLTEEQRNGFIQSLK
DDPSVSKEILAEAKKLNDAQAPKADNKFNKEQQNAFYTILHLPNLTEEQRN
GFIQSLKDDPSVSKEILAEAKKLNDAQAPK
SEQID NO: 83 (E15W)4 
ADNKFNKEQQNAFYWILHLPNLTEEQRNGFIQSLKDDPSVSKEILAEA
KKLNDAQAPKADNKFNKEQQNAFYWILHLPNLTEEQRNGFIQSLKDDPSVS
KEILAEAKKLNDAQAPKADNKFNKEQQNAFYWILHLPNLTEEQRNGFIQSL
KDDPSVSKEILAEAKKLNDAQAPKADNKFNKEQQNAFYWILHLPNLTEEQ
RNGFIQSLKDDPSVSKEILAEAKKLNDAQAPK
SEQ ID NO: 84 (E15L)4 
ADNKFNKEQQNAFYLILHLPNLTEEQRNGFIQSLKDDPSVSKEILAEAK
KLNDAQAPKADNKFNKEQQNAFYLILHLPNLTEEQRNGFIQSLKDDPSVSK
EILAEAKKLNDAQAPKADNKFNKEQQNAFYLILHLPNLTEEQRNGFIQSLK
DDPSVSKEILAEAKKLNDAQAPKADNKFNKEQQNAFYLILHLPNLTEEQRN
GFIQSLKDDPSVSKEILAEAKKLNDAQAPK
SEQ ID NO: 85 (E15V)4 
ADNKFNKEQQNAFYVILHLPNLTEEQRNGFIQSLKDDPSVSKEILAEAK
KLNDAQAPKADNKFNKEQQNAFYVILHLPNLTEEQRNGFIQSLKDDPSVSK
EILAEAKKLNDAQAPKADNKFNKEQQNAFYVILHLPNLTEEQRNGFIQSLK
DDPSVSKEILAEAKKLNDAQAPKADNKFNKEQQNAFYVILHLPNLTEEQRN
GFIQSLKDDPSVSKEILAEAKKLNDAQAPK
SEQ ID NO: 86 (E15I)4 
ADNKFNKEQQNAFYIILHLPNLTEEQRNGFIQSLKDDPSVSKEILAEAK
KLNDAQAPKADNKFNKEQQNAFYIILHLPNLTEEQRNGFIQSLKDDPSVSKE
ILAEAKKLNDAQAPKADNKFNKEQQNAFYIILHLPNLTEEQRNGFIQSLKDD
PSVSKEILAEAKKLNDAQAPKADNKFNKEQQNAFYIILHLPNLTEEQRNGFI
QSLKDDPSVSKEILAEAKKLNDAQAPK
SEQ ID NO: 87 (E15F)4 
ADNKFNKEQQNAFYFILHLPNLTEEQRNGFIQSLKDDPSVSKEILAEAK
KLNDAQAPKADNKFNKEQQNAFYFILHLPNLTEEQRNGFIQSLKDDPSVSK
EILAEAKKLNDAQAPKADNKFNKEQQNAFYFILHLPNLTEEQRNGFIQSLK
DDPSVSKEILAEAKKLNDAQAPKADNKFNKEQQNAFYFILHLPNLTEEQRN
GFIQSLKDDPSVSKEILAEAKKLNDAQAPK
SEQ ID NO: 88 (E15S)4 
ADNKFNKEQQNAFYSILHLPNLTEEQRNGFIQSLKDDPSVSKEILAEAK
KLNDAQAPKADNKFNKEQQNAFYSILHLPNLTEEQRNGFIQSLKDDPSVSK
EILAEAKKLNDAQAPKADNKFNKEQQNAFYSILHLPNLTEEQRNGFIQSLK
DDPSVSKEILAEAKKLNDAQAPKADNKFNKEQQNAFYSILHLPNLTEEQRN
GFIQSLKDDPSVSKEILAEAKKLNDAQAPK
SEQ ID NO: 89 (E15Y)4 
ADNKFNKEQQNAFYYILHLPNLTEEQRNGFIQSLKDDPSVSKEILAEAK
KLNDAQAPKADNKFNKEQQNAFYYILHLPNLTEEQRNGFIQSLKDDPSVSK
EILAEAKKLNDAQAPKADNKFNKEQQNAFYYILHLPNLTEEQRNGFIQSLK
DDPSVSKEILAEAKKLNDAQAPKADNKFNKEQQNAFYYILHLPNLTEEQRN
GFIQSLKDDPSVSKEILAEAKKLNDAQAPK
SEQ ID NO: 90 (E15D)4 
ADNKFNKEQQNAFYDILHLPNLTEEQRNGFIQSLKDDPSVSKEILAEAK
KLNDAQAPKADNKFNKEQQNAFYDILHLPNLTEEQRNGFIQSLKDDPSVSK
EILAEAKKLNDAQAPKADNKFNKEQQNAFYDILHLPNLTEEQRNGFIQSLK
DDPSVSKEILAEAKKLNDAQAPKADNKFNKEQQNAFYDILHLPNLTEEQRN
GFIQSLKDDPSVSKEILAEAKKLNDAQAPK
SEQ ID NO: 91 (Q9I,E15W)4 
ADNKFNKEIQNAFYWILHLPNLTEEQRNGFIQSLKDDPSVSKEILAEAK
KLNDAQAPKADNKFNKEIQNAFYWILHLPNLTEEQRNGFIQSLKDDPSVSK
EILAEAKKLNDAQAPKADNKFNKEIQNAFYWILHLPNLTEEQRNGFIQSLK
DDPSVSKEILAEAKKLNDAQAPKADNKFNKEIQNAFYWILHLPNLTEEQRN
GFIQSLKDDPSVSKEILAEAKKLNDAQAPK
SEQ ID NO: 92 (Q9I,E15L)4 
ADNKFNKEIQNAFYLILHLPNLTEEQRNGFIQSLKDDPSVSKEILAEAK
KLNDAQAPKADNKFNKEIQNAFYLILHLPNLTEEQRNGFIQSLKDDPSVSKE
ILAEAKKLNDAQAPKADNKFNKEIQNAFYLILHLPNLTEEQRNGFIQSLKDD
PSVSKEILAEAKKLNDAQAPKADNKFNKEIQNAFYLILHLPNLTEEQRNGFI
QSLKDDPSVSKEILAEAKKLNDAQAPK
SEQ ID NO: 93 (Q9I,E15V)4 
ADNKFNKEIQNAFYVILHLPNLTEEQRNGFIQSLKDDPSVSKEILAEAK
KLNDAQAPKADNKFNKEIQNAFYVILHLPNLTEEQRNGFIQSLKDDPSVSKE
ILAEAKKLNDAQAPKADNKFNKEIQNAFYVILHLPNLTEEQRNGFIQSLKDD
PSVSKEILAEAKKLNDAQAPKADNKFNKEIQNAFYVILHLPNLTEEQRNGFI
QSLKDDPSVSKEILAEAKKLNDAQAPK
SEQ ID NO: 94 (Q9I,E15I)4 
ADNKFNKEIQNAFYIILHLPNLTEEQRNGFIQSLKDDPSVSKEILAEAKK
LNDAQAPKADNKFNKEIQNAFYIILHLPNLTEEQRNGFIQSLKDDPSVSKEIL
AEAKKLNDAQAPKADNKFNKEIQNAFYIILHLPNLTEEQRNGFIQSLKDDPS
VSKEILAEAKKLNDAQAPKADNKFNKEIQNAFYIILHLPNLTEEQRNGFIQSL
KDDPSVSKEILAEAKKLNDAQAPK
SEQ ID NO: 95 (Q9I,E15F)4 
ADNKFNKEIQNAFYFILHLPNLTEEQRNGFIQSLKDDPSVSKEILAEAKK
LNDAQAPKADNKFNKEIQNAFYFILHLPNLTEEQRNGFIQSLKDDPSVSKEIL
AEAKKLNDAQAPKADNKFNKEIQNAFYFILHLPNLTEEQRNGFIQSLKDDPS
VSKEILAEAKKLNDAQAPKADNKFNKEIQNAFYFILHLPNLTEEQRNGFIQS
LKDDPSVSKEILAEAKKLNDAQAPK
SEQ ID NO: 96 (N3L,N6L,Q9I,E15W)4 
ADLKFLKEIQNAFYWILHLPNLTEEQRNGFIQSLKDDPSVSKEILAEAK
KLNDAQAPKADLKFLKEIQNAFYWILHLPNLTEEQRNGFIQSLKDDPSVSKE
ILAEAKKLNDAQAPKADLKFLKEIQNAFYWILHLPNLTEEQRNGFIQSLKDD
PSVSKEILAEAKKLNDAQAPKADLKFLKEIQNAFYWILHLPNLTEEQRNGFI
QSLKDDPSVSKEILAEAKKLNDAQAPK
SEQ ID NO: 97 (N3L,N6L,Q9I,E15L)4 
ADLKFLKEIQNAFYLILHLPNLTEEQRNGFIQSLKDDPSVSKEILAEAKK
LNDAQAPKADLKFLKEIQNAFYLILHLPNLTEEQRNGFIQSLKDDPSVSKEIL
AEAKKLNDAQAPKADLKFLKEIQNAFYLILHLPNLTEEQRNGFIQSLKDDPS
VSKEILAEAKKLNDAQAPKADLKFLKEIQNAFYLILHLPNLTEEQRNGFIQS
LKDDPSVSKEILAEAKKLNDAQAPK
SEQ ID NO: 98 (N3Y,N6E,Q9I,E15I)4 
ADYKFEKEIQNAFYIILHLPNLTEEQRNGFIQSLKDDPSVSKEILAEAKK
LNDAQAPKADYKFEKEIQNAFYIILHLPNLTEEQRNGFIQSLKDDPSVSKEIL
AEAKKLNDAQAPKADYKFEKEIQNAFYIILHLPNLTEEQRNGFIQSLKDDPS
VSKEILAEAKKLNDAQAPKADYKFEKEIQNAFYIILHLPNLTEEQRNGFIQSL
KDDPSVSKEILAEAKKLNDAQAPK
SEQ ID NO: 99 (N3V,N6L,Q9I,E15V)4 
ADVKFLKEIQNAFYVILHLPNLTEEQRNGFIQSLKDDPSVSKEILAEAK
KLNDAQAPKADVKFLKEIQNAFYVILHLPNLTEEQRNGFIQSLKDDPSVSKE
ILAEAKKLNDAQAPKADVKFLKEIQNAFYVILHLPNLTEEQRNGFIQSLKDD
PSVSKEILAEAKKLNDAQAPKADVKFLKEIQNAFYVILHLPNLTEEQRNGFI
QSLKDDPSVSKEILAEAKKLNDAQAPK
SEQ ID NO: 100 (N3V,N6E,Q9I,E15F)4 
ADVKFEKEIQNAFYFILHLPNLTEEQRNGFIQSLKDDPSVSKEILAEAKK
LNDAQAPKADVKFEKEIQNAFYFILHLPNLTEEQRNGFIQSLKDDPSVSKEIL
AEAKKLNDAQAPKADVKFEKEIQNAFYFILHLPNLTEEQRNGFIQSLKDDPS
VSKEILAEAKKLNDAQAPKADVKFEKEIQNAFYFILHLPNLTEEQRNGFIQS
 LKDDPSVSKEILAEAKKLNDAQAPK
SEQ ID NO: 101 (N3Y,N6E,Q9I,E15L)4 
ADYKFEKEIQNAFYLILHLPNLTEEQRNGFIQSLKDDPSVSKEILAEAKK
LNDAQAPKADYKFEKEIQNAFYLILHLPNLTEEQRNGFIQSLKDDPSVSKEIL
AEAKKLNDAQAPKADYKFEKEIQNAFYLILHLPNLTEEQRNGFIQSLKDDPS
VSKEILAEAKKLNDAQAPKADYKFEKEIQNAFYLILHLPNLTEEQRNGFIQS
LKDDPSVSKEILAEAKKLNDAQAPK
SEQ ID NO: 102 (N3L,N6L,Q9F,E15I)4 
ADLKFLKEFQNAFYIILHLPNLTEEQRNGFIQSLKDDPSVSKEILAEAKK
LNDAQAPKADLKFLKEFQNAFYIILHLPNLTEEQRNGFIQSLKDDPSVSKEIL
AEAKKLNDAQAPKADLKFLKEFQNAFYIILHLPNLTEEQRNGFIQSLKDDPS
VSKEILAEAKKLNDAQAPKADLKFLKEFQNAFYIILHLPNLTEEQRNGFIQSL
KDDPSVSKEILAEAKKLNDAQAPK
SEQ ID NO: 103 (N3V,N6E,Q9I,E15L)4 
ADVKFEKEIQNAFYLILHLPNLTEEQRNGFIQSLKDDPSVSKEILAEAKK
LNDAQAPKADVKFEKEIQNAFYLILHLPNLTEEQRNGFIQSLKDDPSVSKEIL
AEAKKLNDAQAPKADVKFEKEIQNAFYLILHLPNLTEEQRNGFIQSLKDDPS
VSKEILAEAKKLNDAQAPKADVKFEKEIQNAFYLILHLPNLTEEQRNGFIQS
LKDDPSVSKEILAEAKKLNDAQAPK
SEQ ID NO: 104 (N3V,N6E,Q9I,E15I)4 
ADVKFEKEIQNAFYIILHLPNLTEEQRNGFIQSLKDDPSVSKEILAEAKK
LNDAQAPKADVKFEKEIQNAFYIILHLPNLTEEQRNGFIQSLKDDPSVSKEIL
AEAKKLNDAQAPKADVKFEKEIQNAFYIILHLPNLTEEQRNGFIQSLKDDPS
VSKEILAEAKKLNDAQAPKADVKFEKEIQNAFYIILHLPNLTEEQRNGFIQSL
KDDPSVSKEILAEAKKLNDAQAPK
SEQ ID NO: 105 (N3L,N6L,Q9I,E15L)6 
ADLKFLKEIQNAFYLILHLPNLTEEQRNGFIQSLKDDPSVSKEILAEAKK
LNDAQAPKADLKFLKEIQNAFYLILHLPNLTEEQRNGFIQSLKDDPSVSKEIL
 
AEAKKLNDAQAPKADLKFLKEIQNAFYLILHLPNLTEEQRNGFIQSLKDDPS
VSKEILAEAKKLNDAQAPKADLKFLKEIQNAFYLILHLPNLTEEQRNGFIQS
LKDDPSVSKEILAEAKKLNDAQAPKADLKFLKEIQNAFYLILHLPNLTEEQR
NGFIQSLKDDPSVSKEILAEAKKLNDAQAPKADLKFLKEIQNAFYLILHLPN
LTEEQRNGFIQSLKDDPSVSKEILAEAKKLNDAQAPK
SEQ ID NO: 106 (N3L,N6E,Q9I,E15I)6 
ADLKFEKEIQNAFYIILHLPNLTEEQRNGFIQSLKDDPSVSKEILAEAKK
LNDAQAPKADLKFEKEIQNAFYIILHLPNLTEEQRNGFIQSLKDDPSVSKEIL
AEAKKLNDAQAPKADLKFEKEIQNAFYIILHLPNLTEEQRNGFIQSLKDDPS
VSKEILAEAKKLNDAQAPKADLKFEKEIQNAFYIILHLPNLTEEQRNGFIQSL
KDDPSVSKEILAEAKKLNDAQAPKADLKFEKEIQNAFYIILHLPNLTEEQRN
GFIQSLKDDPSVSKEILAEAKKLNDAQAPKADLKFEKEIQNAFYIILHLPNLT
EEQRNGFIQSLKDDPSVSKEILAEAKKLNDAQAPK
SEQ ID NO: 107 (N3V,N6L,Q9I,E15V)6 
ADVKFLKEIQNAFYVILHLPNLTEEQRNGFIQSLKDDPSVSKEILAEAK
KLNDAQAPKADVKFLKEIQNAFYVILHLPNLTEEQRNGFIQSLKDDPSVSKE
ILAEAKKLNDAQAPKADVKFLKEIQNAFYVILHLPNLTEEQRNGFIQSLKDD
PSVSKEILAEAKKLNDAQAPKADVKFLKEIQNAFYVILHLPNLTEEQRNGFI
QSLKDDPSVSKEILAEAKKLNDAQAPKADVKFLKEIQNAFYVILHLPNLTEE
QRNGFIQSLKDDPSVSKEILAEAKKLNDAQAPKADVKFLKEIQNAFYVILHL
PNLTEEQRNGFIQSLKDDPSVSKEILAEAKKLNDAQAPK
SEQ ID NO: 108 (N3L,N6E,Q9F,E15L)6

ADLKFEKEFQNAFYLILHLPNLTEEQRNGFIQSLKDDPSVSKEILAEAK
KLNDAQAPKADLKFEKEFQNAFYLILHLPNLTEEQRNGFIQSLKDDPSVSKE
ILAEAKKLNDAQAPKADLKFEKEFQNAFYLILHLPNLTEEQRNGFIQSLKDD
PSVSKEILAEAKKLNDAQAPKADLKFEKEFQNAFYLILHLPNLTEEQRNGFI
QSLKDDPSVSKEILAEAKKLNDAQAPKADLKFEKEFQNAFYLILHLPNLTEE
QRNGFIQSLKDDPSVSKEILAEAKKLNDAQAPKADLKFEKEFQNAFYLILHL

PNLTEEQRNGFIQSLKDDPSVSKEILAEAKKLNDAQAPK

The present description will be described below in the form of specific examples. It should be understood that these examples are merely illustrative rather than intended to limit the scope of the present description. Unless otherwise specified, the methods and materials used in the following examples are conventional in the art, and some reagents can be obtained from commercial sources.

### Preparation example

### ① Optimization of C domain mutants

Entrust Bioengineering (Shanghai) Co., Ltd. for gene synthesis, the specific steps were as follows:
Constructing the required primers according to the designed sequences. The PCR reaction uses pfu high temperature polymerase.
Dosage of each component of PCR: the concentration of primer is 10D, dissolved in 400 µl ddH₂O.
Reaction system: 50 µl

| | |
|---|---|
| P1: | 252 bp |
| Primers: | 4 µl |
| 10X pfu buffer | 5 µl |
| DNTP | 1 µl (25 mM each) |
| Primers | 2 µl |
| Pfu | 0.4 µl (5 µ/µl) |
| ddH₂O | Replenish water to 50 µl |
| First round of PCR: | |
| 95°C 3min | |
| 95°C 30sec | 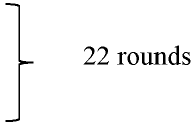 |
| 55°C 30sec | |

| | |
|---|---|
| 72°C | 30sec |
| 72°C | 5min |

The PCR product of the first round used as a template to directly amplify the second round:
P2: 740bp

| | |
|---|---|
| P1 | 2 µl |
| PCR product | 4 µl |
| 10X pfu buffer | 5 µl |
| DNTP | 1 µl (25 mM each) |
| Pfu | 0.4 µl (5 µ/µl) |
| ddH₂O | Replenish water to 50 µl |

Second round of PCR:

| | |
|---|---|
| 95°C 3min | 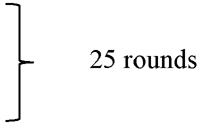 |
| 95°C 30sec | |
| 55°C 30sec | |
| 72°C 50sec | |
| 72°C 5min | |

The products amplified by PCR were subjected to 1% agarose-1X TAE electrophoresis.

The PCR product P2 was recovered and purified by Sangon Biotech's B610353-1000 (EZ-10 Column DNA Gel Recovery Kit). Set for later use.
Clone construction
Linearization enzyme digestion treatment of the vector:
Reaction system: 50 µl

| | |
|---|---|
| pET28a+ | 2 µg |
| 10X FD buffer | 5 µl |
| BamHI | 1 µl (10 µ/µl) |
| HindIII | 1 µl (10 µ/µl) |
| ddH₂O | add water to 50 µl |

The above system was placed in a constant temperature water bath at 37°C for 1 h.

PCR product fragment digestion system, 50 ul:

| | |
|---|---|
| P2 | 1 ug (20 ul) |
| 10X FD buffer | 5 µl |
| BamHI | 1 µl (10 µ/µl) |
| HindIII | 1 µl (10 µ/µl) |
| ddH₂O | add water to 50 µl |

The above system was placed in a constant temperature water bath at 37°C for 2 hours.

The double-digested product was subjected to 1% agarose-1X TAE electrophoresis, and the digested product P2 and vector V (pET28a+) was recovered and purified by Sangon Biotech's B610353-1000 (EZ-10 column DNA gel recovery kit) and set for later use.

The recovered and purified target DNA fragment P2 and the vector V were connected in the T4 DNAligase system.
Ligation system: 20 ul

| | |
|---|---|
| P2 (BamHI/HindIII): | 50 ng |
| pET28a+ (BamHI/HindIII): | 30 ng |
| 10X T4 DNAligase buffer: | 2 ul |
| T4 DNAligase: | 1 ul (5 u/ul) (Thermo Scientific) |
| ddH₂O | added to 20 µl |

The above ligation solution was placed at 16°C for 1 hour.

The above ligation solution was transferred into BL21(DE3) competent cells, and positive clones were detected and screened for sequencing.

### (2) Expression and purification of C domain mutants

Single recombinant plasmids were used to transform into *Escherichia coli,* and cells were fermented and expressed in LB liquid medium by induction. After fermentation, the bacteria were collected and the cell wall was destroyed by thermal lysis to release the expression products, which were separated by centrifugation. The separated liquid was purified through the IgG affinity medium. The separated liquid was loaded on the affinity medium, the medium was washed with 10mM phosphate buffer, and the target protein was collected with a buffer solution of pH 3.8, and adjusted to a neutral environment, stored for later use.

### ③Preparation of affinity chromatography medium from C domain mutants

The obtained C domain mutant was used to prepare an affinity chromatography medium by a conventional method. An exemplary preparation process specifically includes the following steps:
a. Activation
   10 g of high rigidity agarose was taken, washed with purified water and drained. 10g of the above agarose was weighed, 20mL of purified water was added, 0.2g of sodium hydroxide and 10mL of epichlorohydrin were added into a 100mL flask, the above mixture was reacted at a constant temperature of 27°C for 2 hours, the activated gel was washed with 1L of water to obtain activated agarose microspheres.
b. Cross-linking
   The activated agarose microspheres were added to a 100 ml flask, then 150 mg NaHCO₃, 10 mg Na₂CO₃, 150 mg NaCl and 10 mg EDTA were added, 50 ml recombinant protein A solution was added after stirring evenly, the above mixture reacted at a constant temperature of 34°C for 8 hours to complete cross-linking.
c. Blocking
   100 mL of blocking solution (ethanolamine solution) was added to the crosslinked product, the pH was adjusted to 8.6 with sodium hydroxide, and the above mixture was reacted at a constant temperature of 26°C for 2 hours to complete the blocking of the remaining epoxy groups and reduce the impact.

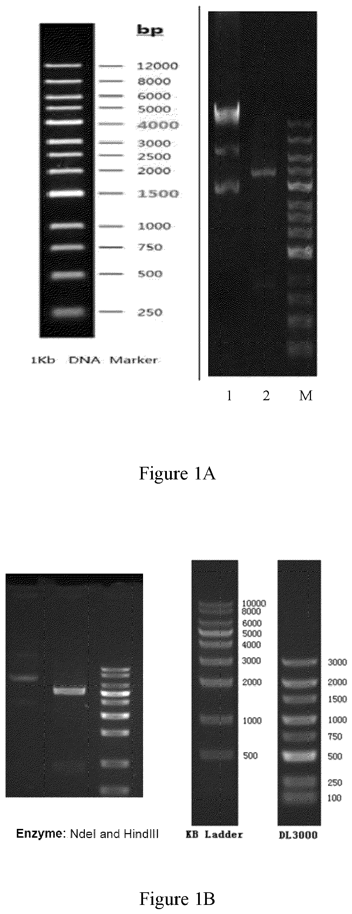

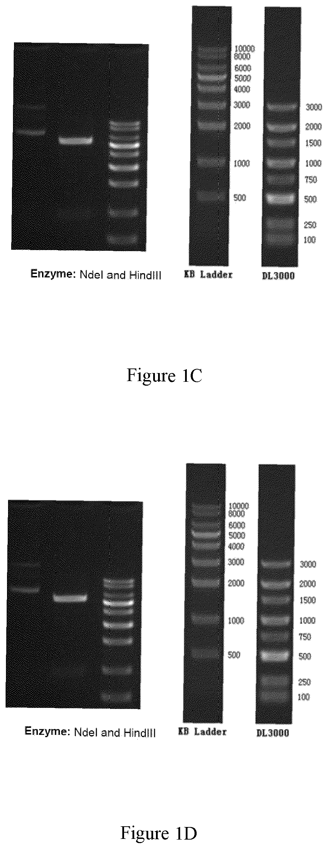

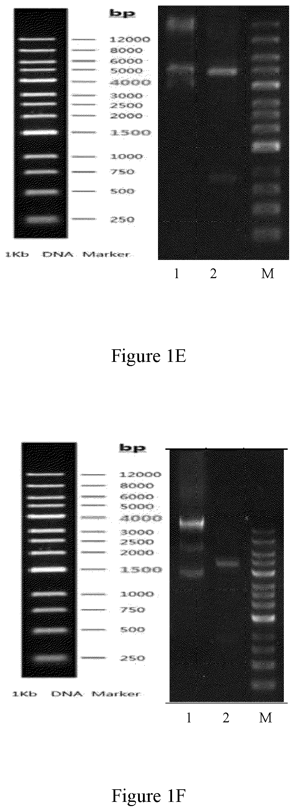

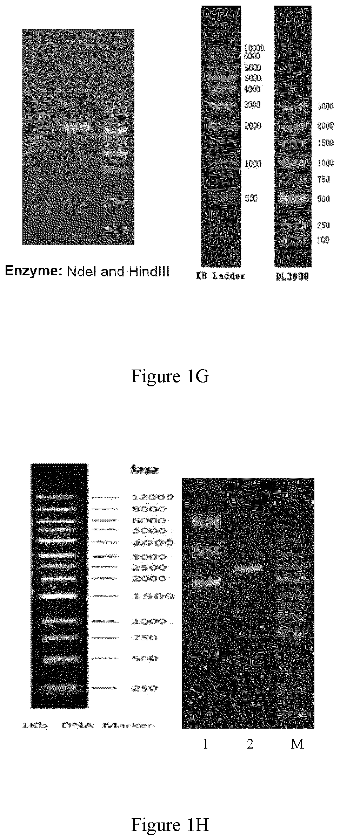

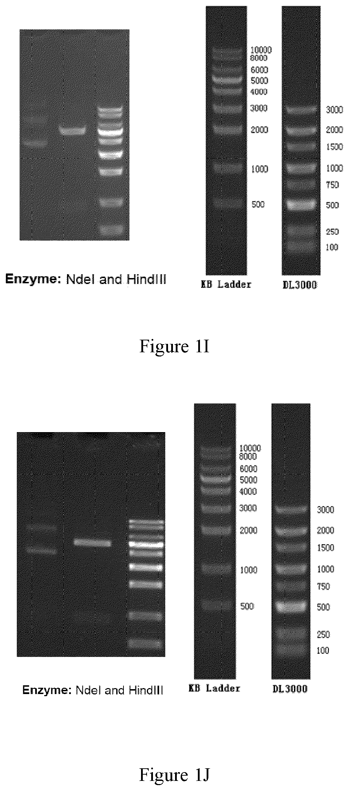

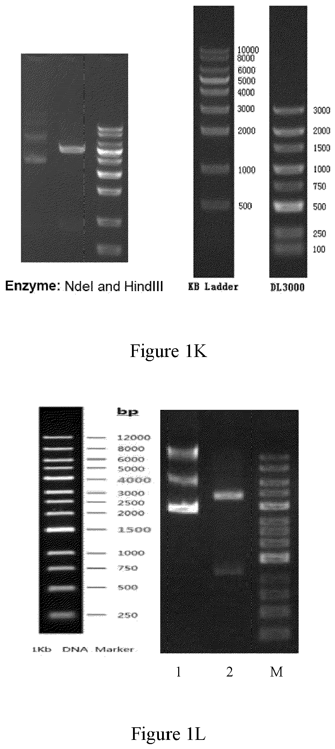

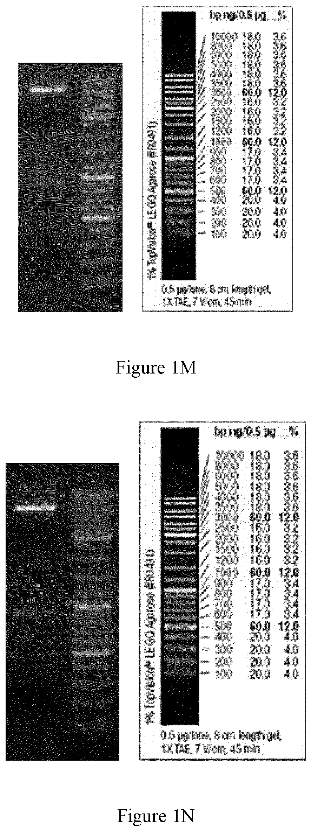

The C domain mutant of the present description was prepared by the above method. Figures 1A-1N show the result analysis diagrams of the C domain mutants in SEQ ID NO: 74-80, 84-87, 101, 103 and 104.

### Example 1

Kinetic analysis of the C domain mutants in Table 1 was performed using the method described below.

The experimental steps are as follows:
1) Cross-linking the Series S CM5 chip with IgG.
a. IgG was diluted to 5 µg/ml, 10 µg/ml, 15 µg/ml, 20 µg/ml with 10 mM acetic acid-NaOH (pH 5.0).
b. Running buffer (10mM acetic acid-NaOH, pH5.0), desorption solution 1 (0.5% SDS), desorption solution 2 (50mM glycine-NaOH, pH9.5), blocking solution (1M ethanolamine pH8.5), activator (0.4M EDC, 0.1M NHS) were prepared.
c. Immobilization was conducted in Biacore-4000.
d. The cross-linking results are shown in the table below:

| Flow cell | Detectio n point | Activation method and time | Usage | Ligand concentration and name | Contact time | Unit (RU) |
|---|---|---|---|---|---|---|
| Fc-2 | 1 | Amino activation for 10 minutes | immobiliz ation | 20 µg/ml IgG | 5 min | 12400 |
| | 2 | Amino activation for 10 minutes | immobiliz ation | 15 µg/ml IgG | 5 min | 7339 |
| | 3 | Amino activation for 10 minutes | Unfixed | / | 0 | 0 |
| | 4 | Amino activation for 10 minutes | immobiliz ation | 10 µg/ml IgG | 5 min | 4294 |
| | 5 | Amino activation for 10 minutes | immobiliz ation | 5 µg/ml IgG | 5 min | 2904 |

2) Kinetic analysis
a. The protein sample (Protein A) was diluted with 1XPBS (pH7.6) to 0 µg/ml, 1.615 µg/ml, 2.4375 µg/ml, 3.25 µg/ml, 4.875 µg/ml, 6.5 µg/ml, 9.75 µg/ml, 13 µg/ml, 19.5 µg/ml, 26 µg/ml.
b. Gel running buffer 1XPBS (pH7.6), regeneration solution (50mM citric acid-NaOH pH3.0) were prepared.
c. Kinetics and affinity were conduct4ed in Biacore-4000.

| Contact time | Flow rate | Dissociation time | Regeneration time |
|---|---|---|---|
| 150 s | 30 µl/min | 250 s | 120 s |

The experimental results are as follows:

**Table 1: Kinetic analysis results of C domain mutants using Biacore**

| C-domain mutant | SEQ ID NO | Ka | Kd | KD |
|---|---|---|---|---|
| (Native C-domain) 4 | **74** | 8.359×10⁴ | 6.000×10⁻⁴ | 7.178×10⁻⁹ |
| **(N3L)4** | 75 | 8.449×10⁴ | 1.937×10⁻⁴ | 2.293×10⁻⁹ |
| **(N3V)4** | 76 | 8.377×10⁴ | 2.949×10⁻⁴ | 3.521×10⁻⁹ |
| **(N6L)4** | 77 | 7.886×10⁴ | 3.333×10⁻⁴ | 4.223×10⁻⁹ |
| **(N6E)4** | 78 | 7.653×10⁴ | 1.916×10⁻⁴ | 2.504×10⁻⁹ |
| **(Q9I)4** | 79 | 1.077×10⁵ | 4.320×10⁻⁴ | 4.013×10⁻⁹ |
| **(Q9F)4** | **80** | 1.999×10⁵ | 9.213×10⁻⁵ | 4.610×10⁻¹⁰ |
| **(Q9M)4** | 81 | 1.145×10⁵ | 3.512×10⁻⁴ | 3.066×10⁻⁹ |
| **(E15T)4** | 82 | 7.480×10⁴ | 3.039×10⁻⁴ | 4.062×10⁻⁹ |
| **(E15W)4** | **83** | 6.296×10⁴ | 4.776×10⁻⁴ | 7.585×10⁻⁹ |
| **(E15L)4** | 84 | 9.494×10⁴ | 4.256×10⁻⁴ | 4.483×10⁻⁹ |
| **(E15V)4** | 85 | 8.683×10⁴ | 1.050×10⁻³ | 1.209×10⁻⁸ |
| **(E15I)4** | **86** | 7.925×10⁴ | 6.603×10⁻⁴ | 8.366×10⁻⁹ |
| **(E15F)4** | **87** | 6.061×10⁴ | 3.166×10⁻⁴ | 5.223×10⁻⁹ |
| **(E15S)4** | **88** | 7.344×10⁴ | 4.061×10⁻⁴ | 5.530×10⁻⁹ |
| **(E15Y)4** | **89** | 9.042×10⁴ | 3.991×10⁻⁴ | 4.414×10⁻⁹ |
| **(E15D)4** | **90** | 8.227×10⁴ | 2.961×10⁻⁴ | 3.599×10⁻⁹ |
| **(Q9I,E15L)4** | **92** | 8.815×10⁴ | 2.693×10⁻⁴ | 3.055×10⁻⁹ |
| **(Q9I,E15V)4** | **93** | 9.403×10⁴ | 1.254×10⁻⁴ | 1.333×10⁻⁹ |
| **(Q9I,E15I)4** | **94** | 9.564×10⁴ | 2.641×10⁻⁴ | 2.762×10⁻⁹ |
| **(N3L,N6L,Q9I,E15W)4** | **96** | 4.354×10⁴ | 3.274×10⁻⁴ | 7.521×10⁻⁹ |
| **(N3L,N6L,Q9I,E15L)4** | **97** | 1.128×10⁵ | 7.298×10⁻⁴ | 6.470×10⁻⁹ |
| **(N3Y,N6E,Q9I,E15I)4** | **98** | 1.669×10⁵ | 9.530×10⁻⁴ | 5.709×10⁻⁹ |
| **(N3V,N6L,Q9I,E15V)4** | **99** | 1.761×10⁵ | 7.727×10⁻⁴ | 4.502×10⁻⁹ |
| **(N3V,N6E,Q9I,E15F)4** | **100** | 1.177×10⁵ | 6.642×10⁻⁴ | 5.644×10⁻⁹ |
| **(N3Y,N6E,Q9I,E15L)4** | **101** | 1.555×10⁵ | 4.934×10⁻⁴ | 3.172×10⁻⁹ |
| **(N3L,N6L,Q9F,E15I)4** | **102** | 8.109×10⁴ | 3.266×10⁻⁴ | 4.027×10⁻⁹ |
| **(N3V,N6E,Q9I,E15L)4** | **103** | 1.240×10⁵ | 2.720×10⁻⁴ | 2.193×10⁻⁹ |
| **(N3V,N6E,Q9I,E15I)4** | **104** | 1.579×10⁵ | 2.241×10⁻⁴ | 1.419×10⁻⁹ |
| **(N3L,N6L,Q9I,E15L)6** | **105** | 1.322×10⁵ | 3.083×10⁻⁴ | 2.331×10⁻⁹ |
| **(N3L,N6E,Q9I,E15I)6** | **106** | 1.135×10⁵ | 4.541×10⁻⁴ | 3.977×10⁻⁹ |
| **(N3V,N6L,Q9I,E15V)6** | **107** | 1.051×10⁵ | 5.933×10⁻⁴ | 5.648×10⁻⁹ |

The results showed that compared with the native C domain, the KD values of all mutants were basically on the same order of magnitude, and the dissociation constant does not change significantly, indicating that the binding ability to IgG of the mutants in the present description, obtained by mutating the C domain, has not been affected.

### Example 2

The IgG binding ability and alkali resistance stability of the C domain mutant affinity chromatography medium in Table 2 were evaluated using the method described below.

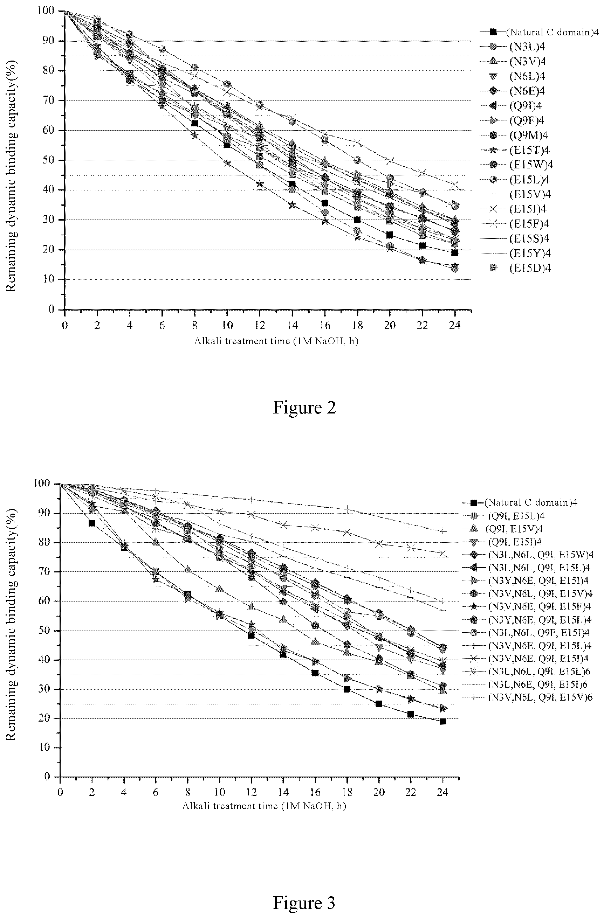

The experimental steps are as follows:
1) 4.4mL of the C domain mutant affinity chromatography medium was taken and packed into the chromatography column with 20% ethanol at a flow rate of 5 ml/min.
   Buffers: Buffer A (PBS, pH 7.6); Buffer B (0.1M Sodium Citrate, pH 3.0); Buffer C (1M NaOH).
   Sample: 1 mg/ml of IgG sample was prepared with the pure product (the solution for diluting the pure product is buffer A).
2) dynamic binding capacity @ 10% breakthrough determination:
   Equipment: AKTA Pure.
   Determination process:
      a. Buffer A equilibrated chromatography column, flow rate: 1.8 ml/min;
      b. When the IGg sample does not flow through the column, its UV absorption peak was detected, calculated as λₘₐₓ, and 10% λₘₐₓ was calculated accordingly;
      c. Sample loading: flow rate: 1.8 ml/min, the UV absorption peak of the basic flow-through was recorded;
      d. The two was added up to get the UV absorption peak corresponding to dynamic binding capacity @ 10% breakthrough;
      e. Keep loading the sample until the UV absorption peak reaches the volume corresponding to the UV absorption peak in step d, which is dynamic binding capacity @ 10% break-through.
3) Alkali resistance cycle:
   The room temperature was controlled at 23±0.5°C; the sample loading volume in the program was updated according to the decrease of binding capacity, and the other programs were kept consistent.
   Determination process:
      a. PBS equilibration: flow rate: 1.8 ml/min; volume: 1 cv;
      b. Sample loading: flow rate: 1.8 ml/min; 200 ml (in subsequent procedures, the sample volume was updated according to the decrease of the binding capacity);
      c. PBS cleaning: flow rate: 1.8 ml/min; volume: 5 cv;
      d.0.1M sodium citrate elution: flow rate: 1.8 ml/min; volume: 3 cv;
      e. Process with 1M NaOH for 2 hours: first using 1.8 ml/min flow rate to pass 1 cv to fill the chromatography column with 1M NaOH, then change flow rate to: 0.5 ml/min; volume: 60 ml; time: 2 h;
      f. PBS equilibration: flow rate: 1.8 ml/min; volume: 10 cv;
4) The above alkali treatment procedure was carried out 13 times, the corresponding binding capacity was recorded, and a chart was drawn. The results were shown in Table 2 and Figure 2) Table 2 shows the DBC of IgG(in terms of dynamic binding capacity, mg/ml) at 10% breakthrough (Qb10%) for each sample.

**Table 2: Affinity chromatography medium with C domain mutants evaluated in column (1M NaOH)**

| C-domain mutant | SEQ ID NO | Initial IgG capability (mg/ml) | Remaining IgG capacity after 24 hours (mg/ml) | Remaining IgG capacity after 24 hours (%) |
|---|---|---|---|---|
| (Native C-domain) 4 | **74** | **165** | **31.28** | **18.96** |
| **(N3L)4** | 75 | 201.28 | 25.52 | 13.64 |
| **(N3V)4** | 76 | 174.68 | 52.57 | 30.10 |
| **(N6L)4** | 77 | 146.32 | 32.38 | 22.13 |
| **(N6E)4** | 78 | 186.02 | 48.62 | 26.14 |
| **(Q9I)4** | 79 | 172.47 | 49.12 | 28.48 |
| **(Q9F)4** | **80** | 145.66 | 51.25 | 35.18 |
| **(Q9M)4** | 81 | 143.29 | 37.82 | 26.39 |
| **(E15T)4** | 82 | 130.8 | 19.08 | 14.59 |
| **(E15W)4** | **83** | 166.24 | 38.64 | 23.24 |
| **(E15L)4** | 84 | 169.66 | 58.48 | 34.47 |
| **(E15V)4** | 85 | 133.01 | 39.99 | 30.07 |
| **(E15I)4** | **86** | 165.59 | 69.32 | 41.86 |
| **(E15F)4** | **87** | 163.51 | 38.48 | 23.53 |
| **(E15S)4** | **88** | 157.02 | 45.9 | 29.23 |
| **(E15Y)4** | **89** | 191.21 | 45.45 | 23.77 |
| **(E15D)4** | **90** | 173.67 | 35.39 | 22.05 |

The results show that the mutation sites selected in the present description can improve the alkaline stability of the native C domain. Among them, (Q9F)4 still has 35.18% of IgG dynamic binding ability after 24 hours of alkali treatment, (E15I)4 still has 41.86% of IgG dynamic binding ability after 24 hours of alkali treatment.

### Example 3

The affinity chromatography medium in table 3 was obtained by C domain mutants, which was processed repeatedly by approach in Example 2, the results were shown in Table 3 and FIG. 3. Table 3 shows the DBC of IgG (in terms of dynamic binding capacity, mg/ml) at 10% breakthrough (Qb10%) for each sample.

**Table 3: Affinity chromatography medium with C domain mutants evaluated in column (1M NaOH)**

| C-domain mutant | SEQ ID NO | Initial IgG capability (mg/ml) | Remaining IgG capacity after 24 hours (mg/ml) | Remaining IgG capacity after 24 hours (%) |
|---|---|---|---|---|
| (Native C-domain) 4 | **74** | **165** | **31.28** | **18.96** |
| **(Q9I,E15L)4** | **92** | **180.2** | **68.2** | **37.85** |
| **(Q9I,E15V)4** | **93** | 147.29 | 43.11 | 29.26 |
| **(Q9I,E15I)4** | **94** | **163.8** | **60.53** | **36.95** |
| **(N3L,N6L,Q9I,E15W)4** | **96** | **189.15** | **83.36** | **44.07** |
| **(N3L,N6L,Q9I,E15L)4** | **97** | 169.7 | 64.38 | 37.93 |
| **(N3Y,N6E,Q9I,E15I)4** | **98** | 155.94 | 36.66 | 23.51 |
| **(N3V,N6L,Q9I,E15V)4** | **99** | 192.87 | 85.55 | 44.35 |
| **(N3V,N6E,Q9I,E15F)4** | **100** | 163.68 | 37.89 | 23.14 |
| **(N3Y,N6E,Q9I,E15L)4** | **101** | 199.39 | 62.15 | 31.17 |
| **(N3L,N6L,Q9F,E15I)4** | **102** | 189.11 | 82.14 | 43.43 |
| **(N3V,N6E,Q9I,E15L)4** | **103** | 69.88 | 58.49 | 83.70 |
| **(N3V,N6E,Q9I,E15I)4** | **104** | 183.21 | 139.72 | 76.26 |
| **(N3L,N6L,Q9I,E15L)6** | **105** | **157.9** | **62.6** | **39.64** |
| **(N3L,N6E,Q9I,E15I)6** | **106** | 147.45 | 83.68 | 56.75 |
| **(N3V,N6L,Q9I,E15V)6** | **107** | **192.79** | **115.51** | **59.91** |

The results show that the alkali resistance of the mutant as in the present description is greatly improved compared with the native C domain.

## Claims

1. A separated polypeptide, wherein, the polypeptide is selected from:
(1) a polypeptide containing the amino acid sequence shown by SEQ ID NO: 109;
(2) A polypeptide having at least 85% sequence identity with the polypeptide described in (1) and retaining the substitution mutations shown in positions 3, 6, 9 and 15 of SEQ ID NO: 109.

2. The polypeptide according to claim 1, wherein in said SEQ ID NO: 109, the substitution mutations at positions 3, 6, 9 and 15 are respectively selected from:
the substitution mutation at position 3 is that asparagine is mutated to leucine, valine or tyrosine;
the substitution mutation at position 6 is that asparagine is mutated to serine, leucine or glutamate;
the substitution mutation at position 9 is that glutamine is mutated to isoleucine, methionine or phenylalanine;
the substitution mutation at the position 15 is glutamate is mutated to threonine, tryptophan, leucine, valine, isoleucine, phenylalanine, serine, tyrosine or aspartate.

3. The polypeptide according to claim 2, wherein in said SEQ ID NO: 109, the substitution mutations at positions 3, 6, 9 and 15 are respectively selected from:
the substitution mutation at the position 3 is that asparagine is mutated to leucine or valine;
the substitution mutation at the position 6 is that asparagine is mutated to leucine or glutamate;
the substitution mutation at the position 9 is that glutamine is mutated to isoleucine or phenylalanine;
the substitution mutation at the position 15 is that glutamate is mutated to tryptophan, leucine, valine, isoleucine or phenylalanine;

4. The polypeptide according to any one of claims 1 to 3, wherein,
the polypeptide has the substitution mutation at position 3 of SEQ ID NO: 109, and optionally has the substitution mutation at one or more positions selected from the group consisting of position 6, 9 and 15; or
the polypeptide has the substitution mutation at position 6 of SEQ ID NO: 109, and optionally has the substitution mutation at one or more positions selected from the group consisting of position 3, 9 and 15; or
the polypeptide has the substitution mutation at position 9 of SEQ ID NO: 109, and optionally has the substitution mutation at one or more positions selected from the group consisting of position 3, 6 and 15; or
the polypeptide has the substitution mutation at position 15 of SEQ ID NO: 109, and optionally has the substitution mutation at one or more positions selected from the group consisting of position 3, 5 and 9; or
the polypeptide has the substitution mutation at position 9 and 15 of SEQ ID NO: 109, and optionally has the substitution mutation at one or all two positions selected from the group consisting of position 3 and 6; or
the polypeptide has the substitution mutation at position 3, 9 and 15 of SEQ ID NO: 109, and optionally has the substitution mutation at position 6; or
the polypeptide has substitution mutations at positions 3, 6, 9 and 15 of SEQ ID NO: 109; wherein, the substitution mutation at position 3 is N3L or N3V, the substitution mutation at position 6 is N6L or N6E, the substitution mutation at position 9 is Q9I or Q9F, and the substitution mutation at position 16 is E15W, E15V, E151 or E15L.

5. The polypeptide according to claim 1, wherein the amino acid sequence of the polypeptide is as shown in any one of SEQ ID NO: 2-73.

6. A separated polypeptide, wherein, the polypeptide consists of the following (i), (ii) and (iii): (i) the polypeptide according to any one of claims 1 to 5, (ii) one or more coupling elements at the C-terminus or N-terminus of the amino acid sequence of the peptide (i), and optionally (iii) residues from the excised signal transduction sequence.

7. The polypeptide according to claim 6, wherein the coupling element is selected from the group consisting of: cysteine residues, multiple lysine residues and multiple histidine residues; the residues of the excised signal transduction sequence are AQ.

8. A fusion protein, wherein the fusion protein comprises an amino acid sequence fused by 2 to 8 of polypeptides according to any one of claims 1 to 5, wherein the 2 to 8 of polypeptides are different from each other, partially or completely identical, and optionally there is a linker sequence between the polypeptides.

9. The fusion protein according to claim 8, wherein each of the polypeptides contained in the fusion protein comprises at least E15T, E15W, E15L, E15V, E15I, E15F, E15S, E15Y or E15T mutations, preferably each of the polypeptides also has a substitution mutation at one or more of positions 3, 6 and 9; preferably, the substitution mutation at position 3 is N3L or N3V; preferably, the substitution mutation at position 6 is N6L or N6E; preferably, the substitution mutation at position 9 is Q9I or Q9F.

10. The fusion protein according to claim 8, wherein, in the fusion protein, the polypeptide is selected from the group consisting of: SEQ ID NO: 2, 3, 6, 7, 8, 9, 11-19, 21-25, 58-62 and 65-69.

11. The fusion protein according to any one of claims 8 to 10, wherein the C-terminus or N-terminus of the fusion protein further comprises one or more coupling elements and/or residues from the excised signal transduction sequence; preferably, the coupling element is selected from the group consisting of: cysteine residues, multiple lysine residues and multiple histidine residues.

12. The fusion protein according to claim 8, wherein the amino acid sequence of the fusion protein is as shown in any one of SEQ ID NO: 75-108.

13. A recombinant protein A, the C domain of the recombinant protein A is the polypeptide according to any one of claims 1 to 5.

14. A separated nucleic acid molecule, the polynucleotide sequence of the nucleic acid molecule is selected from the group consisting of:
(1) a polynucleotide sequence encoding the polypeptide according to any one of claims 1 to 7, the fusion protein according to any one of claims 8 to 12 or the recombinant protein A according to claim 13;
(2) the complementary sequence of the polynucleotide sequence of (1).

15. A nucleic acid construct, wherein, the nucleic acid construct contains the nucleic acid molecule according to claim 14; preferably, the nucleic acid construct is an expression cassette; more preferably, the nucleic acid construct is an expression vector or cloning vector.

16. An expression system comprising the nucleic acid construct according to claim 15; preferably, the expression system is a host cell.

17. A separation medium, wherein, the separation medium comprises the polypeptide according to any one of claims 1 to 7, the fusion protein according to any one of claims 8 to 12 and/or the recombinant protein A according to claim 13, coupled to a solid support.

18. The separation medium according to claim 17, wherein the polypeptide, the fusion protein or the recombinant protein A is coupled to the solid support through a thioether bond.

19. The separation medium of claim 18, wherein the solid support is selected from:
a polymer comprising a polyhydroxyl group, preferably a polysaccharide, more preferably selected from: dextran, starch, cellulose, pullulan, agar and agarose;
a synthetic polymer, preferably selected from the group consisting of: polyvinyl alcohol, polystyrene, polystyrene divinylbenzene, polyhydroxyalkyl acrylates, polyhydroxyalkyl methacrylates, polyacrylamides and polymethacrylamides; and
a support of inorganic nature, preferably selected from silica and zirconia.

20. A chromatographic column, wherein the chromatographic column contains the separation medium according to any one of claims 17 to 19.

21. A method for separating an Fc-containing protein, wherein the method comprises the steps where bring immunoglobulin-containing sample into contact with the polypeptide according to any one of claims 1 to 7, the fusion protein according to any one of claims 8 to 12, the recombinant protein A according to claim 13, the separation medium according to any one of claims 17 to 19, or the chromatographic column according to claim 20; preferably, the Fc-containing protein is immunoglobulin.

22. The method according to claim 21, wherein, the method comprise:
(1) bringing the sample contains an Fc-containing protein into contact with the separation medium;
(2) washing the separation medium;
(3) eluting the Fc-containing protein from the separation medium;
(4) washing the separation medium;
preferably, the separation medium was washed with 0.1-2.0M or 0.5-1.0M NaOH or KOH solution.

23. Use of the polypeptide according to any one of claims 1 to 7, the fusion protein according to any one of claims 8 to 12, the recombinant protein A according to claim 13 in the separation of Fc-containing proteins, or in the preparation of a separation medium or chromatographic column for separating Fc-containing protein.
